# EUROPEAN PATENT APPLICATION

(11) **EP 3 321 683 A1**
(43) Date of publication of application: **16.05.2018**
(21) Application number: 16821499.7
(22) Date of filing: 08.07.2016
(51) Int. Cl.: G01N 33/53, C12M 1/00, C12N 15/09, C12Q 1/68, G01N 33/50, G01N 33/574

(54) **METHOD FOR EVALUATING LYMPH NODE METASTATIC POTENTIAL OF ENDOMETRIAL CANCER**

(30) Priority: 09.07.2015 JP 2015138104
(71) Applicant: Juntendo Educational Foundation, Tokyo 113-8421 (JP)
(72) Inventor: TERAO, Yasuhisa, Tokyo 113-8421 (JP); YOSHIDA, Emiko, Tokyo 113-8421 (JP); TAKEDA, Satoru, Tokyo 113-8421 (JP); HAYASHIZAKI, Yoshihide, Wako-shi Saitama 351-0198 (JP); ITO, Masayoshi, Wako-shi Saitama 351-0198 (JP); KAWAJI, Hideya, Wako-shi Saitama 351-0198 (JP); TANAKA, Yuji, Wako-shi Saitama 351-0198 (JP)
(74) Representative: Blodig, Wolfgang
(86) International application number: PCT/JP2016/070334
(87) International publication number: WO 2017/007031

(57) **Abstract**

Provided is an approach for discriminating endometrial cancer at a molecular level between endometrial cancer with lymph node metastasis and that without metastasis. A method for assessing lymph node metastasis or lymph node metastatic potential, or prognosis of endometrial cancer, including the step of measuring an expression level of SEMA3D in a biological sample derived from a cancer tissue isolated from an endometrial cancer patient.

SEQUENCE LISTING

<110>  JUNTENDO EDUCATIONAL FOUNDATION
       RIKEN

<120>  METHOD FOR EVALUATING LYMPH NODE METASTASIS ABILITY IN UTERINE

<130>  JUSM0014

<150>  JP 2015-138104
<151>  2015-07-09

<160>  34

<170>  PatentIn version 3.5

<210>  1
<211>  6932
<212>  DNA
<213>  Homo sapiens


<220>
<221>  CDS
<222>  (351)..(2684)

<400>  1
agcttcccgg ctctgccagg gctgccggcg gctccgcttg ctcagccagg gctgcggctc      60
agaggaacgc gcgccggtgc cccgggttgc cgcgccgctg gacctcagcc tgcatcccgt     120
tcccctctcc ctgaaaaggc accaggaaaa gagagaggga gagaagagtt cacagcaggt     180
ctttacactt ggactagaac tgtaacatct gctgtcctgg ttgccaggct tttgaacata     240
cactgttggt tctcctggat cttcacctta ccaactgcag atcttgggac tcatcagcct     300
caataattat attaaattaa caccatttga aagagaacat tgttttcatc atg aat        356
gct aat aaa gat gaa aga ctt aaa gcc aga agc caa gat ttt cac ctt       404
ttt cct gct ttg atg atg cta agc atg acc atg ttg ttt ctt cca gtc       452
act ggc act ttg aag caa aat att cca aga ctc aag cta acc tac aaa       500
gac ttg ctg ctt tca aat agc tgt att ccc ttt ttg ggt tca tca gaa       548
gga ctg gat ttt caa act ctt ctc tta gat gag gaa aga ggc agg ctg       596
ctc ttg gga gcc aaa gac cac atc ttt cta ctc agt ctg gtt gac tta       644
aac aaa aat ttt aag aag att tat tgg cct gct gca aag gaa cgg gtg          692
gaa tta tgt aaa tta gct ggg aaa gat gcc aat aca gaa tgt gca aat          740
ttc atc aga gta ctt cag ccc tat aac aaa act cac ata tat gtg tgt          788
gga act gga gca ttt cat cca ata tgt ggg tat att gat ctt gga gtc          836
tac aag gag gat att ata ttc aaa cta gac aca cat aat ttg gag tct          884
ggc aga ctg aaa tgt cct ttc gat cct cag cag cct ttt gct tca gta          932
atg aca gat gag tac ctc tac tct gga aca gct tct gat ttc ctt ggc          980
aaa gat act gca ttc act cga tcc ctt ggg cct act cat gac cac cac          1028
tac atc aga act gac att tca gag cac tac tgg ctc aat gga gca aaa          1076
ttt att gga act ttc ttc ata cca gac acc tac aat cca gat gat gat          1124
aaa ata tat ttc ttc ttt cgt gaa tca tct caa gaa ggc agt acc tcc          1172
gat aaa acc atc ctt tct cga gtt gga aga gtt tgt aag aat gat gta          1220
gga gga caa cgc agc ctg ata aac aag tgg acg act ttt ctt aag gcc          1268
aga ctg att tgc tca att cct gga agt gat ggg gca gat act tac ttt          1316
gat gag ctt caa gat att tat tta ctc ccc aca aga gat gaa aga aat          1364
cct gta gta tat gga gtc ttt act aca acc agc tcc atc ttc aaa ggc          1412
tct gct gtt tgt gtg tat agc atg gct gac atc aga gca gtt ttt aat      1460
ggt cca tat gct cat aag gaa agt gca gac cat cgt tgg gtg cag tat      1508
gat ggg aga att cct tat cca cgg cct ggt aca tgt cca agc aaa acc      1556
tat gac cca ctg att aag tcc acc cga gat ttt cca gat gat gtc atc      1604
agt ttc ata aag cgg cac tct gtg atg tat aag tcc gta tac cca gtt      1652
gca gga gga cca acg ttc aag aga atc aat gtg gat tac aga ctg aca      1700
cag ata gtg gtg gat cat gtc att gca gaa gat ggc cag tac gat gta      1748
atg ttt ctt gga aca gac att gga act gtc ctc aaa gtt gtc agc att      1796
tca aag gaa aag tgg aat atg gaa gag gta gtg ctg gag gag ttg cag      1844
ata ttc aag cac tca tca atc atc ttg aac atg gaa ttg tct ctg aag      1892
cag caa caa ttg tac att ggt tcc cga gat gga ttg gtt cag ctc tcc      1940
ttg cac aga tgc gac act tat ggg aaa gct tgc gca gac tgt tgt ctt      1988
gcc aga gac ccc tac tgt gcc tgg gat gga aat gca tgc tct cga tat      2036
gct cct act tct aaa agg aga gct aga cgc caa gat gta aaa tat ggc      2084
gac cca atc acc cag tgc tgg gac atc gaa gac agc att agt cat gaa      2132
act gct gat gaa aag gtg att ttt ggc att gaa ttt aac tca acc ttt     2180
ctg gaa tgt ata cct aaa tcc caa caa gca act att aaa tgg tat atc     2228
cag agg tca ggg gat gag cat cga gag gag ttg aag ccc gat gaa aga     2276
atc atc aaa acg gaa tat ggg cta ctg att cga agt ttg cag aag aag     2324
gat tct ggg atg tat tac tgc aaa gcc cag gag cac act ttc atc cac     2372
acc ata gtg aag ctg act ttg aat gtc att gag aat gaa cag atg gaa     2420
aat acc cag agg gca gag cat gag gag ggg aag gtc aag gat cta ttg     2468
gct gag tca cgg ttg aga tac aaa gac tac atc caa atc ctt agc agc     2516
cca aac ttc agc ctc gac cag tac tgc gaa cag atg tgg cac agg gag     2564
aag cgg aga cag aga aac aag ggg ggc cca aag tgg aag cac atg cag     2612
gaa atg aag aag aaa cga aat cga aga cat cac aga gac ctg gat gag     2660
ctc cct aga gct gta gcc acg tag ttttctactt aatttaaaga aaagaattcc     2714
ttacctataa aaacattgcc ttctgttttg tatatccctt atagtaattc ataaatgctt     2774
cccatggagt tttgctaagg cacaagacaa taatctgaat aagacaatat gtgatgaata     2834
taagaaaggg caaaaaattc atttgaacca gttttccaag aacaaatctt gcacaagcaa     2894
agtataagaa ttatcctaaa aatagggggt ttacagttgt aaatgtttta tgttttgagt     2954
tttggaattt attgtcatgt aaatagttga gctaagcaag ccccgaattt gatagtgtat     3014
aaggtgcttt attccctcga atgtccatta agcatggaat ttaccatgca gttgtgctat     3074
gttcttatga acagatatat cattcctatt gagaaccagc taccttgtgg tagggaataa     3134
gaggtcagac acaaattaag acaactccca ttatcaacag gaactttctc agtgagccat     3194
tcactcctgg agaatggtat aggaatttgg agaggtgcat tatttctttc tggccactgg 3254
ggttaaattt agtgtactac aacattgatt tactgaaggg cactaatgtt tcccccagga 3314
tttctattga ctagtcagga gtaacaggtt cacagagaga agttggtgct tagttatgtg 3374
ttttttagag tatatactaa gctctacagg gacagaatgc ttaataaata ctttaataag 3434
atatgggaaa atattttaat aaaacaagga aaacataatg atgtataatg catcctgatg 3494
ggaaggcatg cagatgggat ttgttagaag acagaaggaa agacagccat aaattctggc 3554
tttggggaaa actcatatcc ccatgaaaag gaagaacaat cacaaataaa gtgagagtaa 3614
tgtaatggag ctcttttcac tagggtataa gtagctgcca atttgtaatt catctgttaa 3674
aaaaaatcta gattataaca aactgctagc aaaatctgag gaaacataaa ttcttctgaa 3734
gaatcatagg aagagtagac attttattta taaccaatga tatttcagta tatattttct 3794
ctcttttaaa aaatatttat catactctgt atattatttc tttttactgc ctttattctc 3854
tcctgtatat tggattttgt gattatattt gagtgaatag gagaaaacaa tatataacac 3914
acagagaatt aagaaaatga catttctggg gagtggggat atatatttgt tgaataacag 3974
aacgagtgta aaattttaac aacggaaagg gttaaattaa ctctttgaca tcttcactca 4034
accttttctc attgctgagt taatctgttg taattgtagt attgtttttg taatttaaca 4094
ataaataagc ctgctacatg taaaaagaac caaactcaca atattaacat aaacatcttt 4154
catattttgt tagtactttc aaatgttttc aatttgactt tccctctgaa tatgcatggt 4214
gtgtttcctg tctgtttaag cagaactcac ctttccttct tgtaacacag aacccttagc 4274
cttcttctgt tttgcctttt cacgcccttt atagtgtgaa atgaaaaatt agtcacttcc 4334
tcacatggaa ggcagctttt cagaaaataa cagacattgc tcgtttctca tgcattctac 4394
atatcttgaa agaaaagtct gtgagaaaac cctgtgatta gagggcaact taatgcaaga 4454
tctgtggctc tatgttgaga gcattctctc tctgttattt ttattttatt tgcattgctt 4514
acctatctca aagtagtcaa actgatatat gagattgagt actccctttt gatattatac 4574
tgatgaatat ttgtaggtgt ttcactataa ggaacagcta aggaataatt ttaataaaag 4634
tgaaccagaa caaatcactc atttaaaaag taattcagaa gaacagtgtg gcatgatcag 4694
acttctaatt gaatagcgta acaacagtgt ttgtaattat agatttgctt ggacaaaata 4754
ttccaggaac tcatagcgag ctcaaagcaa ttaagtggga acatttttaa tttaaaaaaa 4814
atttccaaat atttgtgggt ccgacagtaa tgatcaaaat atgaatgact ttggaaaatt 4874
tacatgaagc tcaagtgtta ggattgactt atgaaaataa attttatttc tatccaaatt 4934
tgaatgtcca aaccattttt tagttacttc tttctaatcc tagttattca gacaaaattt 4994
ggaaacttat tttatgacca catctaatat tctggctgct ttggatacaa tactcttgat 5054
ttatgataat tagttaaaat atattaaaaa tattattagt aaaataaaat ttcacacaat 5114
aaaaaacaac atagagtaca catatttata tgtattttta aagataaaga atatctaaaa 5174
tgtgtttttt tcttagcttt ttagttgtct agaacattta gagaaagagt atgaatatat 5234
taaatccaca aacacactat atacttcctc accactgact attttatcaa atttgcctta 5294
aaataatgaa aaagaaaagt atagttaatg gtttcaaata tgctaagaaa ttgactctgc 5354
ggaggggatc ttaaaatgcc tgtttactca tgttttcgta tttttcttgt ctctaatact 5414
tttgtctttg tctgccttgt gtgttctttt ctgaatttca tttcagcaat ttatgctgct 5474
aaatagaatc ccgcatgtct gcattcattt acattcaact tattgtaaat tttgagattt 5534
tatttagaaa tgaattgtct aattaattta tgtagagtcc ttttttccaa agagctcaaa 5594
acactacatc cacattattg acttttggaa accaaccaaa ttacttaaga aatggaaaag 5654
tagtcacaga gaaatttagt aatttgcaaa agccacaaca gtagtggaag aattaaaatt 5714
cataaagtct catcattctg ttggctaaaa taatgctctt tacaatatgc tggctacaaa 5774
atggtcttac tcttgaattt tgcttttggt tcatattttg gttcttcata ttctagtatg 5834
ttttgttcct gctgaatttt acagactcag ctaaacaata agttattagt agtcacttgt 5894
tttcattatc ataattgaat ttccaagaac aacttgatat cattcagctg tccaaaaaat 5954
aataagagca ttcattaaat actgattttc ttaaacttaa atgttgtatt tcatatttat 6014
ttatataata caaatggaaa tcaaaactga aatagcctcc ctttagaatg tcacattttt 6074
cttagaaaac attttggaat tggttaacct tattgggaat ggataattta gaaacaaacc 6134
aaaaaaaaaa aaagatattt gaagtgtttt tataggagat gtatttttat catagtaaat 6194
gatcacaatt ttaaataatt catcttacaa gtcaggtaaa ataaaatcga ttcccagatt 6254
ccatacatta ggagtgcaaa gacaggatca tacacatgac ttatacctct catttaaata 6314
ttttatagta taaagtattt atggaaaggg tttgagatag atgatttttg ggaaagggca 6374
aaatgtgtca gatgccctag ttaatcatgt aatttaattc tggtattgtg agtggttagc 6434
aatcaatttt gaatttataa ttctgctatt ttaaaagaaa tgtagtcttg tagtatgaaa 6494
taattaaaaa ttgttaaatg tatattttgg ttgtattata ataaaatgca aacatgactg 6554
ttctatgatt ataaaaacta ctggtttgtg tgcaaatttt gttttcaaac cccaaataca 6614
gtaaatttta tatataaaat aggactaaag aaaatataag atgcaatatt ttctgaaatg 6674
ttcagtagat gtattttaca ctgtaagtat gcagtaattt catttatgaa agaaaagaat 6734
gtgtcccacc caatttgata ccattatatt gctaagcttt attgtgattt aatttaggtt 6794
ttagtggatt gagataaatg ttattaccac aaataactac ttcttttaaa gttctcaatt 6854
gccatctgat tacttaaaaa tactcatcta catgtttagc tgtattggag tataatttat 6914
taaagagtaa ggtatgga 6932

<210>  2
<211>  777
<212>  PRT
<213>  Homo sapiens

<400>  2

<210>  3
<211>  8966
<212>  DNA
<213>  Homo sapiens

<400>  3
   acggaggagg agaaagtcca gttgctgaag ggcagaattt ccatgcgccc gcagcttcca      60
   agtccagacc acccccgaat tcacctctga caaaattctg gggacgctgg gaacactgaa      120
   tcaacatggg caatgagaac agcacctcgg acaaccagag gactttatca gctcagactc      180
   caaggtccgc gcagccaccc gggaacagtc agaatataaa aaggaagcag caggacacgc      240
   ccggaagccc tgaccacaga gacgcgtcca gcattggcag cgttgggctt ggaggcttct      300
   gcaccgcttc tgagagttct gccagcctgg atccatgcct tgtgtcccca gaggtgactg      360
   agccaaggaa ggacccacag ggagccaggg ggccagaagg ttctttgctg cccagcccac      420
   caccgtccca ggagcgagag cacccctcgt cctccatgcc ctttgccgag tgtcccccgg      480
   aaggttgctt ggcaagtcca gcagcggcac ctgaagatgg tcctcagact cagtctccca      540
   ggagggaacc tgccccaaat gccccaggag acatcgcggc ggcatttccc gctgagaggg      600
   acagctctac tccataccaa gagattgctg ccgtccccag tgctggaaga gagagacagc      660
   cgaaggaaga aggacagaag tcctccttct ccttctccag tggcatcgac cagtcacctg      720
   gaatgtcgcc agtacccctc agagagccaa tgaaggcacc gctgtgtgga gagggggacc      780
   agcctggtgg ttttgagtcc caagagaaag aggctgcagg tggctttccc cctgcagagt      840
   ccaggcaggg ggtggcttct gtgcaagtga cccctgaggc ccctgctgca gcccagcagg      900
   gcacagaaag ctcagcggtc ttggagaagt cccccctaaa acccatggcc ccgatcccac      960
   aagatccagc cccaagagcc tcagacagag aaagaggcca aggggaggcg ccgcctcagt     1020
   atttaacaga tgacttggaa ttcctcaggg cctgccatct ccctaggagc aattcagggg     1080
   ctgccccaga agcagaagtg aatgccgctt cccaggagag ctgccagcag ccagtgggag     1140
   catatctgcc gcacgcagag ctgccctggg gcttgccaag tcctgccctg gtgccagagg     1200
   ctgggggctc tgggaaggag gctctggaca ccattgatgt tcagggtcac ccacagacag     1260
   ggatgcgagg aaccaagccc aatcaagttg tctgtgtggc agcaggcggc cagcccgaag 1320
   ggggtttgcc tgtgagccct gaaccttccc tgctcactcc gactgaggaa gcacatccag 1380
   cttcaagcct cgcttcattc ccagctgctc agattcctat tgctgtagaa gaacctggat 1440
   catcatccag ggaatcagtt tccaaggctg ggatgccagt ttctgcagat gcagccaaag 1500
   aggtggtgga tgcagggttg gtgggactgg agaggcaggt gtcagatctt ggaagcaagg 1560
   gagagcatcc agaaggggac cctggagagg ttcctgcccc atcaccccag gagaggggag 1620
   agcacttgaa cacggagcaa agccatgagg tccaaccagg agtaccaccc cctcctcttc 1680
   ccaaggagca aagccatgag gtccaaccag gagcaccacc ccctcctctt cccaaggcac 1740
   caagtgaaag tgccagaggg ccaccggggc caacggatgg agccaaggtc catgaagatt 1800
   ccacaagccc agccgtggct aaagaaggaa gcagatcacc tggtgacagc cctggaggaa 1860
   aggaggaagc cccagagcca cctgatggtg gagacccagg gaacctgcaa ggagaggact 1920
   ctcaggcttt cagcagcaag cgtgatccag aagtaggcaa agatgagctt tcaaagccaa 1980
   gcagtgatgc agagagcaga gaccatccca gctcacactc agcacagcca cccagaaagg 2040
   ggggtgctgg gcacacggac gggccccact ctcagacagc agaggctgat gcatctggcc 2100
   taccacacaa gctgggtgag gaggaccccg tcctgccccc tgtgccagat ggagctggtg 2160
   agcccactgt tcccgaagga gccatctggg aggggtcagg attgcagccc aaatgtcctg 2220
   acacccttca gagcagggaa ggattgggaa gaatggagtc tttcctgact ttagaatcag 2280
   agaaatcaga ttttccacca actcctgttg cagaggttgc acccaaagcc caggaaggtg 2340
   agagcacatt ggaaataagg aagatgggca gctgtgatgg ggagggcttg ctgacgtccc 2400
   cagatcaacc ccgcgggccg gcgtgtgatg cgtcgagaca ggaatttcat gctggggtgc 2460
   cacatccccc ccagggggag aacttggcag cagacctggg gctcacggca ctcatcctgg 2520
   accaagatca gcagggaatc ccatcctgcc caggggaagg ctggataaga ggagctgcat 2580
   ccgagtggcc cctactatct tctgagaagc atctccagcc atcccaggca caaccagaga 2640
   catccatctt tgacgtgctc aaggagcagg cccagccacc tgaaaatggg aaagagactt 2700
   ctccaagcca tccaggtttt aaggaccagg gagcagattc ttcccaaatc catgtacctg 2760
   tggaacctca ggaagataac aacttgccca ctcatggagg acaggagcag gctttgggat 2820
   cagaacttca aagtcagctc cccaaaggca ccctgtctga tactccaact tcatctccca 2880
   ctgacatggt ttgggagagt tctctgacag aagagtcaga attgtcagca ccaacgagac 2940
   agaagttgcc tgcactaggg gagaagcggc cagagggagc atgcggtgat ggtcagtcct 3000
   cgaggtctcc gcctccagca gcagatgtct taaaagactt ttctcttgca gggaacttca 3060
   gcagaaagga aacttgctgc actgggcagg ggccaaacaa gtctcaacag gcattggctg 3120
   atgccttgga agaaggcagc cagcatgaag aagcatgtca aaggcatcca ggagcttctg 3180
   aagcagctga tggttgttcc ccactctggg gcttgagtaa gagggagatg gcaagtggaa 3240
   acacagggga ggccccacct tgtcagcctg actcagtagc tctcctggat gcagttccct 3300
   gcctgccagc cctggcgccc gccagccccg gagtcacacc cacccaggat gccccagaga 3360
   cagaggcatg tgatgaaacc caggaaggca ggcagcaacc agtgccggcc ccgcagcaga 3420
   aaatggagtg ctgggccact tcggatgcag agtccccaaa gcttcttgca agtttcccat 3480
   cagctgggga gcaaggtggt gaagccgggg ctgctgagac tggtggcagc gctggtgcag 3540
   gagacccagg aaagcagcag gctccggaga aacctggaga agctactttg agttgtggcc 3600
   tccttcagac tgagcactgc cttacctccg gggaggaagc ttctacctct gccctacgtg 3660
   agtcctgcca agctgagcac cccatggcca gctgccagga tgccttgctg ccagccagag 3720
   agctgggtgg gattcccagg agcaccatgg atttttctac acaccaggct gtcccagacc 3780
   caaaggagct cctgctgtct gggccaccag aagtggctgc tcctgacacc ccttacctgc 3840
   atgtcgacag tgctgcccag agaggagcag aagacagtgg agtgaaagct gtttcctctg 3900
   cagaccccag agctcctggc gaaagcccct gtcctgtagg ggagccccca cttgccttgg 3960
   aaaatgctgc ctccttgaag ctgtttgctg gctccctcgc ccccctgttg caaccaggag 4020
   ctgcaggtgg ggaaatccct gcagtgcaag ccagcagtgg tagtcccaaa gccagaacca 4080
   ctgagggacc agtggactcc atgccatgcc tggaccggat gccacttctg gccaagggca 4140
   agcaggcaac aggggaagag aaagcagcaa cagctccagg tgcaggtgcc aaggccagtg 4200
   gggagggcat ggcaggtgat gcagcaggag agacagaggg cagcatggag aggatgggag 4260
   agccttccca ggacccaaag cagggcacat caggtggtgt ggacacaagc tctgagcaaa 4320
   tcgccaccct cactggcttc ccagacttca gggagcacat cgccaagatc ttcgagaagc 4380
   ctgtgctcgg agccctggcc acacctggag aaaaggcagg agctgggagg agtgcagtgg 4440
   gtaaagacct caccaggcca ttgggcccag agaagcttct agatgggcct ccaggagtgg 4500
   atgtcaccct tctccctgca cctcctgctc gactccaggt ggagaagaag caacagttgg 4560
   ctggagaggc tgagatttcc catctggctc tgcaagatcc agcttcagac aagcttctgg 4620
   gtccagcagg gctgacctgg gagcggaact tgccaggtgc cggtgtgggg aaggagatgg 4680
   caggtgtccc acccacactg agggaagacg agaggccaga ggggcctggg gcagcctggc 4740
   caggcctgga aggccaggct tactcacagc tggagaggag caggcaggaa ttagcttcag 4800
   gtcttccttc accagcagct actcaggagc tccctgtgga gagagctgct gccttccagg 4860
   tggctcccca tagccatgga gaagaggccg tggcccaaga cagaattcct tctggaaagc 4920
   agcaccagga aacatctgcc tgcgacagtc cacatggaga agatggtccc ggggactttg 4980
   ctcacacagg ggttccagga catgtgccaa ggtccacgtg tgccccttct cctcagaggg 5040
   aggttttgac tgtgcctgag gccaacagtg agccctggac ccttgacacg cttgggggtg 5100
   aaaggagacc cggagtcact gctggcatct tggaaatgcg aaatgccctg ggcaaccaga 5160
   gcacccctgc accaccaact ggagaagtgg cagacactcc cctggagcct ggcaaggtgg 5220
   caggcgctgc tggggaagca gagggtgaca tcaccctgag cacagctgag acacaggcat 5280
   gtgcgtccgg tgatctgcct gaagcaggta ctacgaggac attctccgtt gtggcaggtg 5340
   acttggtgct gccaggaagc tgtcaggacc cagcctgctc tgacaaggct ccggggatgg 5400
   agggtacagc tgcccttcat ggggacagcc cagccaggcc ccagcaggct aaggagcagc 5460
   cagggcctga gcgccccatt ccagctgggg atgggaaggt gtgcgtctcc tcacctccag 5520
   agcctgacga aactcacgac ccgaagctgc aacatttggc tccagaagag ctccacactg 5580
   acagagagag ccccaggcct ggcccatcca tgttaccttc ggttcctaag aaggatgctc 5640
   caagagtcat ggataaagtc acttcagatg agaccagagg tgcggaagga acagaaagga 5700
   gttccgattc tgaagaggca tttgagaccc cggagtcaac gacccctgtc aaagctccgc 5760
   cagctccacc cccaccaccc cccgaagtca tcccagaacc cgaggtcagc acacagccac 5820
   ccccggaaga accaggatgt ggttctgaga cagtccctgt ccctgatggc ccacggagcg 5880
   actcggtgga aggaagtccc ttccgtcccc cgtcacactc cttctctgcc gtcttcgatg 5940
   aagacaagcc gatagccagc agtgggactt acaacttgga ctttgacaac attgagcttg 6000
   tggatacctt tcagaccttg gagcctcgtg cctcagacgc taagaatcag gagggcaaag 6060
   tgaacacacg gaggaagtcc acggattccg tccccatctc taagtctaca ctgtcccggt 6120
   cgctcagcct gcaagccagt gactttgatg gtgcttcttc ctcaggcaat cccgaggccg 6180
   tggcccttgc cccagatgca tatagcacgg gttccagcag tgcttctagt acccttaagc 6240
   gaactaaaaa accgaggccg ccttccttaa aaaagaaaca gaccaccaag aaacccacag 6300
   agaccccccc agtgaaggag acgcaacagg agccagatga agagagcctt gtccccagtg 6360
   gggagaatct agcatctgag acgaaaacgg aatctgccaa gacggaaggt cctagcccag 6420
   ccttattgga ggagacgccc cttgagcccg ctgtggggcc caaagctgcc tgccctctgg 6480
   actcagagag tgcagaaggg gttgtccccc cggcttctgg aggtggcaga gtgcagaact 6540
   caccccctgt cgggaggaaa acgctgcctc ttaccacggc cccggaggca ggggaggtaa 6600
   ccccatcgga tagcgggggg caagaggact ctccagccaa agggctctcc gtaaggctgg 6660
   agtttgacta ttctgaggac aagagtagtt gggacaacca gcaggaaaac ccccctccta 6720
   ccaaaaagat aggcaaaaag ccagttgcca aaatgcccct gaggaggcca aagatgaaaa 6780
   agacacccga gaaacttgac aacactcctg cctcacctcc cagatcccct gctgaaccca 6840
   atgacatccc cattgctaaa ggtacttaca cctttgatat tgacaagtgg gatgacccca 6900
   attttaaccc tttttcttcc acctcaaaaa tgcaggagtc tcccaaactg ccccaacaat 6960
   catacaactt tgacccagac acctgtgatg agtccgttga cccctttaag acatcctcta 7020
   agacccccag ctcaccttct aaatccccag cctcctttga gatcccagcc agtgctatgg 7080
   aagccaatgg agtggacggg gatgggctaa acaagcccgc caagaagaag aagacgcccc 7140
   taaagactga cacatttagg gtgaaaaagt cgccaaaacg gtctcctctc tctgatccac 7200
   cttcccagga ccccacccca gctgctacac cagaaacacc accagtgatc tctgcggtgg 7260
   tccacgccac agatgaggaa aagctggcgg tcaccaacca gaagtggacg tgcatgacag 7320
   tggacctaga ggctgacaaa caggactacc cgcagccctc ggacctgtcc acctttgtaa 7380
   acgagaccaa attcagttca cccactgagg agttggatta cagaaactcc tatgaaattg 7440
   aatatatgga gaaaattggc tcctccttac ctcaggacga cgatgccccg aagaagcagg 7500
   ccttgtacct tatgtttgac acttctcagg agagccctgt caagtcatct cccgtccgca 7560
   tgtcagagtc cccgacgccg tgttcagggt caagttttga agagactgaa gcccttgtga 7620
   acactgctgc gaaaaaccag catcctgtcc cacgaggact ggcccctaac caagagtcac 7680
   acttgcaggt gccagagaaa tcctcccaga aggagctgga ggccatgggc ttgggcaccc 7740
   cttcagaagc gattgaaatt agagaggctg ctcacccaac agacgtctcc atctccaaaa 7800
   cagccttgta ctcccgcatc gggaccgctg aggtggagaa acctgcaggc cttctgttcc 7860
   agcagcccga cctggactct gccctccaga tcgccagagc agagatcata accaaggaga 7920
   gagaggtctc agaatggaaa gataaatatg aagaaagcag gcgggaagtg atggaaatga 7980
   ggaaaatagt ggccgagtat gagaagacca tcgctcagat gatagaggac gaacagagag 8040
   agaagtcagt ctcccaccag acggtgcagc agctggttct ggagaaggag caagccctgg 8100
   ccgacctgaa ctccgtggag aagtctctgg ccgacctctt cagaagatat gagaagatga 8160
   aggaggtcct agaaggcttc cgcaagaatg aagaggtgtt gaagagatgt gcgcaggagt 8220
   acctgtcccg ggtgaagaag gaggagcaga ggtaccaggc cctgaaggtg cacgcggagg 8280
   agaaactgga cagggccaat gctgagattg ctcaggttcg aggcaaggcc cagcaggagc 8340
   aagccgccca ccaggccagc ctgcggaagg agcagctgcg agtggacgcc ctggaaagga 8400
   cgctggagca gaagaataaa gaaatagaag aactcaccaa gatttgtgac gaactgattg 8460
   ccaaaatggg gaaaagctaa ctctgaaccg aatgttttgg acttaactgt tgcgtgcaat 8520
   atgaccgtcg gcacactgct gttcctccag ttccatggac aggttctgtt ttcacttttt 8580
   cgtatgcact actgtatttc ctttctaaat aaaattgatt tgattgtatg cagtactaag 8640
   gagactatca gaatttcttg ctattggttt gcattttcct agtataattc atagcaagtt 8700
   gacctcagag ttcctgtatc agggagattg tctgattctc taataaaaga cacattgctg 8760
   accttggcct tgccctttgt acacaagttc ccagggtgag cagcttttgg atttaatatg 8820
   aacatgtaca gcgtgcatag ggactcttgc cttaaggagt gtaaacttga tctgcatttg 8880
   ctgatttgtt tttaaaaaaa caagaaatgc atgtttcaaa taaaattctc tattgtaaat 8940
   aaaatttttt ctttggatct tggcaa 8966

<210> 4
<211> 3800
<212> DNA
<213> Homo sapiens

<400> 4
acggaggagg agaaagtcca gttgctgaag ggcagaattt ccatgcgccc gcagcttcca 60

agtccagacc acccccgaat tcacctctga caaaattctg gggacgctgg gaacactgaa 120

tcaacatggg caatgagaac agcacctcgg acaaccagag gactttatca gctcagactc 180

caaggtccgc gcagccaccc gggaacagtc agaatataaa aaggaagcag caggacacgc 240

ccggaagccc tgaccacaga gacgcgtcca gttcacctgt ggcagatgat atcatccagc 300

ccgctgcccc cgcagacctg gaaagcccaa ccttagctgc ctcttcctac cacggtgatg 360

ttgttggcca ggtctctacg gatctgatag cccagagcat ctccccagct gctgcccatg 420

cgggtcttcc tccctcggct gcagaacaca tagtttcgcc atctgcccca gctggtgaca 480

gagtagaagc ttccactccc tcctgcccag atccggccaa ggacctcagc aggagttccg 540

attctgaaga ggcatttgag accccggagt caacgacccc tgtcaaagct ccgccagctc 600

cacccccacc accccccgaa gtcatcccag aacccgaggt cagcacacag ccacccccgg 660

aagaaccagg atgtggttct gagacagtcc ctgtccctga tggcccacgg agcgactcgg 720

tggaaggaag tcccttccgt cccccgtcac actccttctc tgccgtcttc gatgaagaca 780

agccgatagc cagcagtggg acttacaact tggactttga caacattgag cttgtggata 840

cctttcagac cttggagcct cgtgcctcag acgctaagaa tcaggagggc aaagtgaaca 900

cacggaggaa gtccacggat tccgtcccca tctctaagtc tacactgtcc cggtcgctca 960

gcctgcaagc cagtgacttt gatggtgctt cttcctcagg caatcccgag gccgtggccc 1020

ttgccccaga tgcatatagc acgggttcca gcagtgcttc tagtaccctt aagcgaacta 1080

aaaaaccgag gccgccttcc ttaaaaaaga aacagaccac caagaaaccc acagagaccc 1140

ccccagtgaa ggagacgcaa caggagccag atgaagagag ccttgtcccc agtggggaga 1200

atctagcatc tgagacgaaa acggaatctg ccaagacgga aggtcctagc ccagccttat 1260

tggaggagac gccccttgag cccgctgtgg ggcccaaagc tgcctgccct ctggactcag 1320

agagtgcaga aggggttgtc cccccggctt ctggaggtgg cagagtgcag aactcacccc 1380

ctgtcgggag gaaaacgctg cctcttacca cggccccgga ggcaggggag gtaaccccat 1440

cggatagcgg ggggcaagag gactctccag ccaaagggct ctccgtaagg ctggagtttg 1500

actattctga ggacaagagt agttgggaca accagcagga aaacccccct cctaccaaaa 1560

agataggcaa aaagccagtt gccaaaatgc ccctgaggag gccaaagatg aaaaagacac 1620

ccgagaaact tgacaacact cctgcctcac ctcccagatc ccctgctgaa cccaatgaca 1680

tccccattgc taaaggtact tacacctttg atattgacaa gtgggatgac cccaatttta 1740

accctttttc ttccacctca aaaatgcagg agtctcccaa actgccccaa caatcataca 1800

actttgaccc agacacctgt gatgagtccg ttgacccctt taagacatcc tctaagaccc 1860

ccagctcacc ttctaaatcc ccagcctcct ttgagatccc agccagtgct atggaagcca 1920

atggagtgga cggggatggg ctaaacaagc ccgccaagaa gaagaagacg cccctaaaga 1980

ctgacacatt tagggtgaaa aagtcgccaa aacggtctcc tctctctgat ccaccttccc 2040

aggaccccac cccagctgct acaccagaaa caccaccagt gatctctgcg gtggtccacg 2100

ccacagatga ggaaaagctg gcggtcacca accagaagtg gacgtgcatg acagtggacc 2160

tagaggctga caaacaggac tacccgcagc cctcggacct gtccaccttt gtaaacgaga 2220

ccaaattcag ttcacccact gaggagttgg attacagaaa ctcctatgaa attgaatata 2280

tggagaaaat tggctcctcc ttacctcagg acgacgatgc cccgaagaag caggccttgt 2340

accttatgtt tgacacttct caggagagcc ctgtcaagtc atctcccgtc cgcatgtcag 2400

agtccccgac gccgtgttca gggtcaagtt ttgaagagac tgaagccctt gtgaacactg 2460

ctgcgaaaaa ccagcatcct gtcccacgag gactggcccc taaccaagag tcacacttgc 2520

aggtgccaga gaaatcctcc cagaaggagc tggaggccat gggcttgggc accccttcag 2580

aagcgattga aattagagag gctgctcacc caacagacgt ctccatctcc aaaacagcct 2640

tgtactcccg catcgggacc gctgaggtgg agaaacctgc aggccttctg ttccagcagc 2700

ccgacctgga ctctgccctc cagatcgcca gagcagagat cataaccaag gagagagagg 2760

tctcagaatg gaaagataaa tatgaagaaa gcaggcggga agtgatggaa atgaggaaaa 2820

tagtggccga gtatgagaag accatcgctc agatgataga ggacgaacag agagagaagt 2880

cagtctccca ccagacggtg cagcagctgg ttctggagaa ggagcaagcc ctggccgacc 2940

tgaactccgt ggagaagtct ctggccgacc tcttcagaag atatgagaag atgaaggagg 3000

tcctagaagg cttccgcaag aatgaagagg tgttgaagag atgtgcgcag gagtacctgt 3060

cccgggtgaa gaaggaggag cagaggtacc aggccctgaa ggtgcacgcg gaggagaaac 3120

tggacagggc caatgctgag attgctcagg ttcgaggcaa ggcccagcag gagcaagccg 3180

cccaccaggc cagcctgcgg aaggagcagc tgcgagtgga cgccctggaa aggacgctgg 3240

agcagaagaa taaagaaata gaagaactca ccaagatttg tgacgaactg attgccaaaa 3300

tggggaaaag ctaactctga accgaatgtt ttggacttaa ctgttgcgtg caatatgacc 3360

gtcggcacac tgctgttcct ccagttccat ggacaggttc tgttttcact ttttcgtatg 3420

cactactgta tttcctttct aaataaaatt gatttgattg tatgcagtac taaggagact 3480

atcagaattt cttgctattg gtttgcattt tcctagtata attcatagca agttgacctc 3540

agagttcctg tatcagggag attgtctgat tctctaataa aagacacatt gctgaccttg 3600

gccttgccct ttgtacacaa gttcccaggg tgagcagctt ttggatttaa tatgaacatg 3660

tacagcgtgc atagggactc ttgccttaag gagtgtaaac ttgatctgca tttgctgatt 3720

tgtttttaaa aaaacaagaa atgcatgttt caaataaaat tctctattgt aaataaaatt 3780

ttttctttgg atcttggcaa 3800

<210> 5
<211> 2948
<212> PRT
<213> Homo sapiens

<400> 5

Met Gly Asn Glu Asn Ser Thr Ser Asp Asn Gln Arg Thr Leu Ser Ala
1               5                   10                  15

Gln Thr Pro Arg Ser Ala Gln Pro Pro Gly Asn Ser Gln Asn Ile Lys
            20                  25                  30

Arg Lys Gln Gln Asp Thr Pro Gly Ser Pro Asp His Arg Asp Ala Ser
        35                  40                  45

Ser Ile Gly Ser Val Gly Leu Gly Gly Phe Cys Thr Ala Ser Glu Ser
    50                  55                  60

Ser Ala Ser Leu Asp Pro Cys Leu Val Ser Pro Glu Val Thr Glu Pro
65                  70                  75                  80

Arg Lys Asp Pro Gln Gly Ala Arg Gly Pro Glu Gly Ser Leu Leu Pro
                85                  90                  95

Ser Pro Pro Pro Ser Gln Glu Arg Glu His Pro Ser Ser Ser Met Pro
            100                 105                 110

Phe Ala Glu Cys Pro Pro Glu Gly Cys Leu Ala Ser Pro Ala Ala Ala
        115                 120                 125

Pro Glu Asp Gly Pro Gln Thr Gln Ser Pro Arg Arg Glu Pro Ala Pro
    130                 135                 140

Asn Ala Pro Gly Asp Ile Ala Ala Ala Phe Pro Ala Glu Arg Asp Ser
145                 150                 155                 160

Ser Thr Pro Tyr Gln Glu Ile Ala Ala Val Pro Ser Ala Gly Arg Glu
                165                 170                 175

Arg Gln Pro Lys Glu Glu Gly Gln Lys Ser Ser Phe Ser Phe Ser Ser
            180                 185                 190

Gly Ile Asp Gln Ser Pro Gly Met Ser Pro Val Pro Leu Arg Glu Pro
        195                 200                 205

Met Lys Ala Pro Leu Cys Gly Glu Gly Asp Gln Pro Gly Gly Phe Glu
    210                 215                 220

Ser Gln Glu Lys Glu Ala Ala Gly Gly Phe Pro Pro Ala Glu Ser Arg
225                 230                 235                 240

Gln Gly Val Ala Ser Val Gln Val Thr Pro Glu Ala Pro Ala Ala Ala
                245                 250                 255

Gln Gln Gly Thr Glu Ser Ser Ala Val Leu Glu Lys Ser Pro Leu Lys
            260                 265                 270

Pro Met Ala Pro Ile Pro Gln Asp Pro Ala Pro Arg Ala Ser Asp Arg
        275                 280                 285

Glu Arg Gly Gln Gly Glu Ala Pro Pro Gln Tyr Leu Thr Asp Asp Leu
    290                 295                 300

Glu Phe Leu Arg Ala Cys His Leu Pro Arg Ser Asn Ser Gly Ala Ala
305                 310                 315                 320

Pro Glu Ala Glu Val Asn Ala Ala Ser Gln Glu Ser Cys Gln Gln Pro
                325                 330                 335

Val Gly Ala Tyr Leu Pro His Ala Glu Leu Pro Trp Gly Leu Pro Ser
            340                 345                 350

Pro Ala Leu Val Pro Glu Ala Gly Gly Ser Gly Lys Glu Ala Leu Asp
        355                 360                 365

Thr Ile Asp Val Gln Gly His Pro Gln Thr Gly Met Arg Gly Thr Lys
    370                 375                 380

Pro Asn Gln Val Val Cys Val Ala Ala Gly Gly Gln Pro Glu Gly Gly
385                 390                 395                 400

Leu Pro Val Ser Pro Glu Pro Ser Leu Leu Thr Pro Thr Glu Glu Ala
                405                 410                 415

His Pro Ala Ser Ser Leu Ala Ser Phe Pro Ala Ala Gln Ile Pro Ile
            420                 425                 430

Ala Val Glu Glu Pro Gly Ser Ser Ser Arg Glu Ser Val Ser Lys Ala
        435                 440                 445

Gly Met Pro Val Ser Ala Asp Ala Ala Lys Glu Val Val Asp Ala Gly
    450                 455                 460

Leu Val Gly Leu Glu Arg Gln Val Ser Asp Leu Gly Ser Lys Gly Glu
465                 470                 475                 480

His Pro Glu Gly Asp Pro Gly Glu Val Pro Ala Pro Ser Pro Gln Glu
                485                 490                 495

Arg Gly Glu His Leu Asn Thr Glu Gln Ser His Glu Val Gln Pro Gly
            500                 505                 510

Val Pro Pro Pro Pro Leu Pro Lys Glu Gln Ser His Glu Val Gln Pro
        515                 520                 525

Gly Ala Pro Pro Pro Pro Leu Pro Lys Ala Pro Ser Glu Ser Ala Arg
    530                 535                 540

Gly Pro Pro Gly Pro Thr Asp Gly Ala Lys Val His Glu Asp Ser Thr
545                 550                 555                 560

Ser Pro Ala Val Ala Lys Glu Gly Ser Arg Ser Pro Gly Asp Ser Pro
                565                 570                 575

Gly Gly Lys Glu Glu Ala Pro Glu Pro Pro Asp Gly Gly Asp Pro Gly
            580                 585                 590

Asn Leu Gln Gly Glu Asp Ser Gln Ala Phe Ser Ser Lys Arg Asp Pro
        595                 600                 605

Glu Val Gly Lys Asp Glu Leu Ser Lys Pro Ser Ser Asp Ala Glu Ser
    610                 615                 620

Arg Asp His Pro Ser Ser His Ser Ala Gln Pro Pro Arg Lys Gly Gly
625                 630                 635                 640

Ala Gly His Thr Asp Gly Pro His Ser Gln Thr Ala Glu Ala Asp Ala
                645                 650                 655

Ser Gly Leu Pro His Lys Leu Gly Glu Glu Asp Pro Val Leu Pro Pro

            660                 665                 670

Val Pro Asp Gly Ala Gly Glu Pro Thr Val Pro Glu Gly Ala Ile Trp
        675                 680                 685

Glu Gly Ser Gly Leu Gln Pro Lys Cys Pro Asp Thr Leu Gln Ser Arg
    690                 695                 700

Glu Gly Leu Gly Arg Met Glu Ser Phe Leu Thr Leu Glu Ser Glu Lys
705                 710                 715                 720

Ser Asp Phe Pro Pro Thr Pro Val Ala Glu Val Ala Pro Lys Ala Gln
                725                 730                 735

Glu Gly Glu Ser Thr Leu Glu Ile Arg Lys Met Gly Ser Cys Asp Gly
            740                 745                 750

Glu Gly Leu Leu Thr Ser Pro Asp Gln Pro Arg Gly Pro Ala Cys Asp
        755                 760                 765

Ala Ser Arg Gln Glu Phe His Ala Gly Val Pro His Pro Pro Gln Gly
    770                 775                 780

Glu Asn Leu Ala Ala Asp Leu Gly Leu Thr Ala Leu Ile Leu Asp Gln
785                 790                 795                 800

Asp Gln Gln Gly Ile Pro Ser Cys Pro Gly Glu Gly Trp Ile Arg Gly
                805                 810                 815

Ala Ala Ser Glu Trp Pro Leu Leu Ser Ser Glu Lys His Leu Gln Pro
            820                 825                 830

Ser Gln Ala Gln Pro Glu Thr Ser Ile Phe Asp Val Leu Lys Glu Gln
        835                 840                 845

Ala Gln Pro Pro Glu Asn Gly Lys Glu Thr Ser Pro Ser His Pro Gly
    850                 855                 860

Phe Lys Asp Gln Gly Ala Asp Ser Ser Gln Ile His Val Pro Val Glu
865                 870                 875                 880

Pro Gln Glu Asp Asn Asn Leu Pro Thr His Gly Gly Gln Glu Gln Ala
                885                 890                 895

Leu Gly Ser Glu Leu Gln Ser Gln Leu Pro Lys Gly Thr Leu Ser Asp
            900                 905                 910

Thr Pro Thr Ser Ser Pro Thr Asp Met Val Trp Glu Ser Ser Leu Thr
        915                 920                 925

Glu Glu Ser Glu Leu Ser Ala Pro Thr Arg Gln Lys Leu Pro Ala Leu
    930                 935                 940

Gly Glu Lys Arg Pro Glu Gly Ala Cys Gly Asp Gly Gln Ser Ser Arg
945                 950                 955                 960

Val Ser Pro Pro Ala Ala Asp Val Leu Lys Asp Phe Ser Leu Ala Gly
                965                 970                 975

Asn Phe Ser Arg Lys Glu Thr Cys Cys Thr Gly Gln Gly Pro Asn Lys
            980                 985                 990

Ser Gln Gln Ala Leu Ala Asp Ala Leu Glu Glu Gly Ser Gln His Glu
        995                 1000                1005

Glu Ala Cys Gln Arg His Pro Gly Ala Ser Glu Ala Ala Asp Gly
    1010                1015                1020

Cys Ser Pro Leu Trp Gly Leu Ser Lys Arg Glu Met Ala Ser Gly
    1025                1030                1035

Asn Thr Gly Glu Ala Pro Pro Cys Gln Pro Asp Ser Val Ala Leu
    1040                1045                1050

Leu Asp Ala Val Pro Cys Leu Pro Ala Leu Ala Pro Ala Ser Pro
    1055                1060                1065

Gly Val Thr Pro Thr Gln Asp Ala Pro Glu Thr Glu Ala Cys Asp
    1070                1075                1080

Glu Thr Gln Glu Gly Arg Gln Gln Pro Val Pro Ala Pro Gln Gln
    1085                1090                1095

Lys Met Glu Cys Trp Ala Thr Ser Asp Ala Glu Ser Pro Lys Leu
    1100                1105                1110

Leu Ala Ser Phe Pro Ser Ala Gly Glu Gln Gly Gly Glu Ala Gly
    1115                1120                1125

Ala Ala Glu Thr Gly Gly Ser Ala Gly Ala Gly Asp Pro Gly Lys
    1130                1135                1140

Gln Gln Ala Pro Glu Lys Pro Gly Glu Ala Thr Leu Ser Cys Gly
    1145                1150                1155

Leu Leu Gln Thr Glu His Cys Leu Thr Ser Gly Glu Glu Ala Ser
    1160                1165                1170

Thr Ser Ala Leu Arg Glu Ser Cys Gln Ala Glu His Pro Met Ala
    1175                1180                1185

Ser Cys Gln Asp Ala Leu Leu Pro Ala Arg Glu Leu Gly Gly Ile
    1190                1195                1200

Pro Arg Ser Thr Met Asp Phe Ser Thr His Gln Ala Val Pro Asp
    1205                1210                1215

Pro Lys Glu Leu Leu Leu Ser Gly Pro Pro Glu Val Ala Ala Pro
    1220                1225                1230

Asp Thr Pro Tyr Leu His Val Asp Ser Ala Ala Gln Arg Gly Ala
    1235                1240                1245

Glu Asp Ser Gly Val Lys Ala Val Ser Ser Ala Asp Pro Arg Ala
    1250                1255                1260

Pro Gly Glu Ser Pro Cys Pro Val Gly Glu Pro Pro Leu Ala Leu
    1265                1270                1275

Glu Asn Ala Ala Ser Leu Lys Leu Phe Ala Gly Ser Leu Ala Pro
    1280                1285                1290

Leu Leu Gln Pro Gly Ala Ala Gly Gly Glu Ile Pro Ala Val Gln
    1295                1300                1305

Ala Ser Ser Gly Ser Pro Lys Ala Arg Thr Thr Glu Gly Pro Val
    1310                1315                1320

Asp Ser Met Pro Cys Leu Asp Arg Met Pro Leu Leu Ala Lys Gly
    1325                1330                1335

Lys Gln Ala Thr Gly Glu Glu Lys Ala Ala Thr Ala Pro Gly Ala
    1340                1345                1350

Gly Ala Lys Ala Ser Gly Glu Gly Met Ala Gly Asp Ala Ala Gly
    1355                1360                1365

Glu Thr Glu Gly Ser Met Glu Arg Met Gly Glu Pro Ser Gln Asp
    1370                1375                1380

Pro Lys Gln Gly Thr Ser Gly Gly Val Asp Thr Ser Ser Glu Gln
    1385                1390                1395

Ile Ala Thr Leu Thr Gly Phe Pro Asp Phe Arg Glu His Ile Ala
    1400                1405                1410

Lys Ile Phe Glu Lys Pro Val Leu Gly Ala Leu Ala Thr Pro Gly
    1415                1420                1425

Glu Lys Ala Gly Ala Gly Arg Ser Ala Val Gly Lys Asp Leu Thr
    1430                1435                1440

Arg Pro Leu Gly Pro Glu Lys Leu Leu Asp Gly Pro Pro Gly Val
    1445                1450                1455

Asp Val Thr Leu Leu Pro Ala Pro Pro Ala Arg Leu Gln Val Glu
    1460                1465                1470

Lys Lys Gln Gln Leu Ala Gly Glu Ala Glu Ile Ser His Leu Ala
    1475                1480                1485

Leu Gln Asp Pro Ala Ser Asp Lys Leu Leu Gly Pro Ala Gly Leu
    1490                1495                1500

Thr Trp Glu Arg Asn Leu Pro Gly Ala Gly Val Gly Lys Glu Met
    1505                1510                1515

Ala Gly Val Pro Pro Thr Leu Arg Glu Asp Glu Arg Pro Glu Gly
    1520                1525                1530

Pro Gly Ala Ala Trp Pro Gly Leu Glu Gly Gln Ala Tyr Ser Gln
    1535                1540                1545

Leu Glu Arg Ser Arg Gln Glu Leu Ala Ser Gly Leu Pro Ser Pro
    1550                1555                1560

Ala Ala Thr Gln Glu Leu Pro Val Glu Arg Ala Ala Ala Phe Gln
    1565                1570                1575

Val Ala Pro His Ser His Gly Glu Glu Ala Val Ala Gln Asp Arg
    1580                1585                1590

Ile Pro Ser Gly Lys Gln His Gln Glu Thr Ser Ala Cys Asp Ser
    1595                1600                1605

Pro His Gly Glu Asp Gly Pro Gly Asp Phe Ala His Thr Gly Val
    1610                1615                1620

Pro Gly His Val Pro Arg Ser Thr Cys Ala Pro Ser Pro Gln Arg

1625                1630                1635

Glu Val Leu Thr Val Pro Glu Ala Asn Ser Glu Pro Trp Thr Leu
    1640                1645                1650

Asp Thr Leu Gly Gly Glu Arg Arg Pro Gly Val Thr Ala Gly Ile
    1655                1660                1665

Leu Glu Met Arg Asn Ala Leu Gly Asn Gln Ser Thr Pro Ala Pro
    1670                1675                1680

Pro Thr Gly Glu Val Ala Asp Thr Pro Leu Glu Pro Gly Lys Val
    1685                1690                1695

Ala Gly Ala Ala Gly Glu Ala Glu Gly Asp Ile Thr Leu Ser Thr
    1700                1705                1710

Ala Glu Thr Gln Ala Cys Ala Ser Gly Asp Leu Pro Glu Ala Gly
    1715                1720                1725

Thr Thr Arg Thr Phe Ser Val Val Ala Gly Asp Leu Val Leu Pro
    1730                1735                1740

Gly Ser Cys Gln Asp Pro Ala Cys Ser Asp Lys Ala Pro Gly Met
    1745                1750                1755

Glu Gly Thr Ala Ala Leu His Gly Asp Ser Pro Ala Arg Pro Gln
    1760                1765                1770

Gln Ala Lys Glu Gln Pro Gly Pro Glu Arg Pro Ile Pro Ala Gly
    1775                1780                1785

Asp Gly Lys Val Cys Val Ser Ser Pro Pro Glu Pro Asp Glu Thr
    1790                1795                1800

His Asp Pro Lys Leu Gln His Leu Ala Pro Glu Glu Leu His Thr
    1805                1810                1815

Asp Arg Glu Ser Pro Arg Pro Gly Pro Ser Met Leu Pro Ser Val
    1820                1825                1830

Pro Lys Lys Asp Ala Pro Arg Val Met Asp Lys Val Thr Ser Asp
    1835                1840                1845

Glu Thr Arg Gly Ala Glu Gly Thr Glu Ser Ser Pro Val Ala Asp
    1850                1855                1860

Asp Ile Ile Gln Pro Ala Ala Pro Ala Asp Leu Glu Ser Pro Thr
    1865                1870                1875

Leu Ala Ala Ser Ser Tyr His Gly Asp Val Val Gly Gln Val Ser
    1880                1885                1890

Thr Asp Leu Ile Ala Gln Ser Ile Ser Pro Ala Ala Ala His Ala
    1895                1900                1905

Gly Leu Pro Pro Ser Ala Ala Glu His Ile Val Ser Pro Ser Ala
    1910                1915                1920

Pro Ala Gly Asp Arg Val Glu Ala Ser Thr Pro Ser Cys Pro Asp
    1925                1930                1935

Pro Ala Lys Asp Leu Ser Arg Ser Ser Asp Ser Glu Glu Ala Phe
    1940                1945                1950

Glu Thr Pro Glu Ser Thr Thr Pro Val Lys Ala Pro Pro Ala Pro
    1955                1960                1965

Pro Pro Pro Pro Pro Glu Val Ile Pro Glu Pro Glu Val Ser Thr
    1970                1975                1980

Gln Pro Pro Pro Glu Glu Pro Gly Cys Gly Ser Glu Thr Val Pro
    1985                1990                1995

Val Pro Asp Gly Pro Arg Ser Asp Ser Val Glu Gly Ser Pro Phe
    2000                2005                2010

Arg Pro Pro Ser His Ser Phe Ser Ala Val Phe Asp Glu Asp Lys
    2015                2020                2025

Pro Ile Ala Ser Ser Gly Thr Tyr Asn Leu Asp Phe Asp Asn Ile
    2030                2035                2040

Glu Leu Val Asp Thr Phe Gln Thr Leu Glu Pro Arg Ala Ser Asp
    2045                2050                2055

Ala Lys Asn Gln Glu Gly Lys Val Asn Thr Arg Arg Lys Ser Thr
    2060                2065                2070

Asp Ser Val Pro Ile Ser Lys Ser Thr Leu Ser Arg Ser Leu Ser
    2075                2080                2085

Leu Gln Ala Ser Asp Phe Asp Gly Ala Ser Ser Ser Gly Asn Pro
    2090                2095                2100

Glu Ala Val Ala Leu Ala Pro Asp Ala Tyr Ser Thr Gly Ser Ser
    2105                2110                2115

Ser Ala Ser Ser Thr Leu Lys Arg Thr Lys Lys Pro Arg Pro Pro
    2120                2125                2130

Ser Leu Lys Lys Lys Gln Thr Thr Lys Lys Pro Thr Glu Thr Pro
    2135                2140                2145

Pro Val Lys Glu Thr Gln Gln Glu Pro Asp Glu Glu Ser Leu Val
    2150                2155                2160

Pro Ser Gly Glu Asn Leu Ala Ser Glu Thr Lys Thr Glu Ser Ala
    2165                2170                2175

Lys Thr Glu Gly Pro Ser Pro Ala Leu Leu Glu Glu Thr Pro Leu
    2180                2185                2190

Glu Pro Ala Val Gly Pro Lys Ala Ala Cys Pro Leu Asp Ser Glu
    2195                2200                2205

Ser Ala Glu Gly Val Val Pro Pro Ala Ser Gly Gly Gly Arg Val
    2210                2215                2220

Gln Asn Ser Pro Pro Val Gly Arg Lys Thr Leu Pro Leu Thr Thr
    2225                2230                2235

Ala Pro Glu Ala Gly Glu Val Thr Pro Ser Asp Ser Gly Gly Gln
    2240                2245                2250

Glu Asp Ser Pro Ala Lys Gly Leu Ser Val Arg Leu Glu Phe Asp
    2255                2260                2265

Tyr Ser Glu Asp Lys Ser Ser Trp Asp Asn Gln Gln Glu Asn Pro
    2270                2275                2280

Pro Pro Thr Lys Lys Ile Gly Lys Lys Pro Val Ala Lys Met Pro
    2285                2290                2295

Leu Arg Arg Pro Lys Met Lys Lys Thr Pro Glu Lys Leu Asp Asn
    2300                2305                2310

Thr Pro Ala Ser Pro Pro Arg Ser Pro Ala Glu Pro Asn Asp Ile
    2315                2320                2325

Pro Ile Ala Lys Gly Thr Tyr Thr Phe Asp Ile Asp Lys Trp Asp
    2330                2335                2340

Asp Pro Asn Phe Asn Pro Phe Ser Ser Thr Ser Lys Met Gln Glu
    2345                2350                2355

Ser Pro Lys Leu Pro Gln Gln Ser Tyr Asn Phe Asp Pro Asp Thr
    2360                2365                2370

Cys Asp Glu Ser Val Asp Pro Phe Lys Thr Ser Ser Lys Thr Pro
    2375                2380                2385

Ser Ser Pro Ser Lys Ser Pro Ala Ser Phe Glu Ile Pro Ala Ser
    2390                2395                2400

Ala Met Glu Ala Asn Gly Val Asp Gly Asp Gly Leu Asn Lys Pro
    2405                2410                2415

Ala Lys Lys Lys Lys Thr Pro Leu Lys Thr Asp Thr Phe Arg Val
    2420                2425                2430

Lys Lys Ser Pro Lys Arg Ser Pro Leu Ser Asp Pro Pro Ser Gln
    2435                2440                2445

Asp Pro Thr Pro Ala Ala Thr Pro Glu Thr Pro Pro Val Ile Ser
    2450                2455                2460

Ala Val Val His Ala Thr Asp Glu Glu Lys Leu Ala Val Thr Asn
    2465                2470                2475

Gln Lys Trp Thr Cys Met Thr Val Asp Leu Glu Ala Asp Lys Gln
    2480                2485                2490

Asp Tyr Pro Gln Pro Ser Asp Leu Ser Thr Phe Val Asn Glu Thr
    2495                2500                2505

Lys Phe Ser Ser Pro Thr Glu Glu Leu Asp Tyr Arg Asn Ser Tyr
    2510                2515                2520

Glu Ile Glu Tyr Met Glu Lys Ile Gly Ser Ser Leu Pro Gln Asp
    2525                2530                2535

Asp Asp Ala Pro Lys Lys Gln Ala Leu Tyr Leu Met Phe Asp Thr
    2540                2545                2550

Ser Gln Glu Ser Pro Val Lys Ser Ser Pro Val Arg Met Ser Glu
    2555                2560                2565

Ser Pro Thr Pro Cys Ser Gly Ser Ser Phe Glu Glu Thr Glu Ala

2570                2575                2580

Leu Val Asn Thr Ala Ala Lys Asn Gln His Pro Val Pro Arg Gly
    2585                2590                2595

Leu Ala Pro Asn Gln Glu Ser His Leu Gln Val Pro Glu Lys Ser
    2600                2605                2610

Ser Gln Lys Glu Leu Glu Ala Met Gly Leu Gly Thr Pro Ser Glu
    2615                2620                2625

Ala Ile Glu Ile Thr Ala Pro Glu Gly Ser Phe Ala Ser Ala Asp
    2630                2635                2640

Ala Leu Leu Ser Arg Leu Ala His Pro Val Ser Leu Cys Gly Ala
    2645                2650                2655

Leu Asp Tyr Leu Glu Pro Asp Leu Ala Glu Lys Asn Pro Pro Leu
    2660                2665                2670

Phe Ala Gln Lys Leu Gln Glu Glu Leu Glu Phe Ala Ile Met Arg
    2675                2680                2685

Ile Glu Ala Leu Lys Leu Ala Arg Gln Ile Ala Leu Ala Ser Arg
    2690                2695                2700

Ser His Gln Asp Ala Lys Arg Glu Ala Ala His Pro Thr Asp Val
    2705                2710                2715

Ser Ile Ser Lys Thr Ala Leu Tyr Ser Arg Ile Gly Thr Ala Glu
    2720                2725                2730

Val Glu Lys Pro Ala Gly Leu Leu Phe Gln Gln Pro Asp Leu Asp
    2735                2740                2745

Ser Ala Leu Gln Ile Ala Arg Ala Glu Ile Ile Thr Lys Glu Arg
    2750                2755                2760

Glu Val Ser Glu Trp Lys Asp Lys Tyr Glu Glu Ser Arg Arg Glu
    2765                2770                2775

Val Met Glu Met Arg Lys Ile Val Ala Glu Tyr Glu Lys Thr Ile
    2780                2785                2790

Ala Gln Met Ile Glu Asp Glu Gln Arg Glu Lys Ser Val Ser His
    2795                2800                2805

Gln Thr Val Gln Gln Leu Val Leu Glu Lys Glu Gln Ala Leu Ala
    2810                2815                2820

Asp Leu Asn Ser Val Glu Lys Ser Leu Ala Asp Leu Phe Arg Arg
    2825                2830                2835

Tyr Glu Lys Met Lys Glu Val Leu Glu Gly Phe Arg Lys Asn Glu
    2840                2845                2850

Glu Val Leu Lys Arg Cys Ala Gln Glu Tyr Leu Ser Arg Val Lys
    2855                2860                2865

Lys Glu Glu Gln Arg Tyr Gln Ala Leu Lys Val His Ala Glu Glu
    2870                2875                2880

Lys Leu Asp Arg Ala Asn Ala Glu Ile Ala Gln Val Arg Gly Lys
    2885                2890                2895

Ala Gln Gln Glu Gln Ala Ala His Gln Ala Ser Leu Arg Lys Glu
    2900                2905                2910

Gln Leu Arg Val Asp Ala Leu Glu Arg Thr Leu Glu Gln Lys Asn
    2915                2920                2925

Lys Glu Ile Glu Glu Leu Thr Lys Ile Cys Asp Glu Leu Ile Ala
    2930                2935                2940

Lys Met Gly Lys Ser
    2945

<210> 6
<211> 2875
<212> PRT
<213> Homo sapiens

<400> 6

Met Gly Asn Glu Asn Ser Thr Ser Asp Asn Gln Arg Thr Leu Ser Ala
1               5                   10                  15

Gln Thr Pro Arg Ser Ala Gln Pro Pro Gly Asn Ser Gln Asn Ile Lys
            20                  25                  30

Arg Lys Gln Gln Asp Thr Pro Gly Ser Pro Asp His Arg Asp Ala Ser
        35                  40                  45

Ser Ile Gly Ser Val Gly Leu Gly Gly Phe Cys Thr Ala Ser Glu Ser
    50                  55                  60

Ser Ala Ser Leu Asp Pro Cys Leu Val Ser Pro Glu Val Thr Glu Pro
65                  70                  75                  80

Arg Lys Asp Pro Gln Gly Ala Arg Gly Pro Glu Gly Ser Leu Leu Pro
                85                  90                  95

Ser Pro Pro Pro Ser Gln Glu Arg Glu His Pro Ser Ser Ser Met Pro
            100                 105                 110

Phe Ala Glu Cys Pro Pro Glu Gly Cys Leu Ala Ser Pro Ala Ala Ala
        115                 120                 125

Pro Glu Asp Gly Pro Gln Thr Gln Ser Pro Arg Arg Glu Pro Ala Pro
    130                 135                 140

Asn Ala Pro Gly Asp Ile Ala Ala Ala Phe Pro Ala Glu Arg Asp Ser
145                 150                 155                 160

Ser Thr Pro Tyr Gln Glu Ile Ala Ala Val Pro Ser Ala Gly Arg Glu
                165                 170                 175

Arg Gln Pro Lys Glu Glu Gly Gln Lys Ser Ser Phe Ser Phe Ser Ser
            180                 185                 190

Gly Ile Asp Gln Ser Pro Gly Met Ser Pro Val Pro Leu Arg Glu Pro
        195                 200                 205

Met Lys Ala Pro Leu Cys Gly Glu Gly Asp Gln Pro Gly Gly Phe Glu
    210                 215                 220

Ser Gln Glu Lys Glu Ala Ala Gly Gly Phe Pro Pro Ala Glu Ser Arg
225                 230                 235                 240

Gln Gly Val Ala Ser Val Gln Val Thr Pro Glu Ala Pro Ala Ala Ala
                245                 250                 255

Gln Gln Gly Thr Glu Ser Ser Ala Val Leu Glu Lys Ser Pro Leu Lys
            260                 265                 270

Pro Met Ala Pro Ile Pro Gln Asp Pro Ala Pro Arg Ala Ser Asp Arg
        275                 280                 285

Glu Arg Gly Gln Gly Glu Ala Pro Pro Gln Tyr Leu Thr Asp Asp Leu
    290                 295                 300

Glu Phe Leu Arg Ala Cys His Leu Pro Arg Ser Asn Ser Gly Ala Ala
305                 310                 315                 320

Pro Glu Ala Glu Val Asn Ala Ala Ser Gln Glu Ser Cys Gln Gln Pro
                325                 330                 335

Val Gly Ala Tyr Leu Pro His Ala Glu Leu Pro Trp Gly Leu Pro Ser
            340                 345                 350

Pro Ala Leu Val Pro Glu Ala Gly Gly Ser Gly Lys Glu Ala Leu Asp
        355                 360                 365

Thr Ile Asp Val Gln Gly His Pro Gln Thr Gly Met Arg Gly Thr Lys
    370                 375                 380

Pro Asn Gln Val Val Cys Val Ala Ala Gly Gly Gln Pro Glu Gly Gly
385                 390                 395                 400

Leu Pro Val Ser Pro Glu Pro Ser Leu Leu Thr Pro Thr Glu Glu Ala
                405                 410                 415

His Pro Ala Ser Ser Leu Ala Ser Phe Pro Ala Ala Gln Ile Pro Ile
            420                 425                 430

Ala Val Glu Glu Pro Gly Ser Ser Ser Arg Glu Ser Val Ser Lys Ala
        435                 440                 445

Gly Met Pro Val Ser Ala Asp Ala Ala Lys Glu Val Val Asp Ala Gly
    450                 455                 460

Leu Val Gly Leu Glu Arg Gln Val Ser Asp Leu Gly Ser Lys Gly Glu
465                 470                 475                 480

His Pro Glu Gly Asp Pro Gly Glu Val Pro Ala Pro Ser Pro Gln Glu
                485                 490                 495

Arg Gly Glu His Leu Asn Thr Glu Gln Ser His Glu Val Gln Pro Gly
            500                 505                 510

Val Pro Pro Pro Pro Leu Pro Lys Glu Gln Ser His Glu Val Gln Pro
        515                 520                 525

Gly Ala Pro Pro Pro Pro Leu Pro Lys Ala Pro Ser Glu Ser Ala Arg
    530                 535                 540

Gly Pro Pro Gly Pro Thr Asp Gly Ala Lys Val His Glu Asp Ser Thr
545                 550                 555                 560

Ser Pro Ala Val Ala Lys Glu Gly Ser Arg Ser Pro Gly Asp Ser Pro
                565                 570                 575

Gly Gly Lys Glu Glu Ala Pro Glu Pro Pro Asp Gly Gly Asp Pro Gly
            580                 585                 590

Asn Leu Gln Gly Glu Asp Ser Gln Ala Phe Ser Ser Lys Arg Asp Pro
        595                 600                 605

Glu Val Gly Lys Asp Glu Leu Ser Lys Pro Ser Ser Asp Ala Glu Ser
    610                 615                 620

Arg Asp His Pro Ser Ser His Ser Ala Gln Pro Pro Arg Lys Gly Gly
625                 630                 635                 640

Ala Gly His Thr Asp Gly Pro His Ser Gln Thr Ala Glu Ala Asp Ala
                645                 650                 655

Ser Gly Leu Pro His Lys Leu Gly Glu Glu Asp Pro Val Leu Pro Pro
            660                 665                 670

Val Pro Asp Gly Ala Gly Glu Pro Thr Val Pro Glu Gly Ala Ile Trp
        675                 680                 685

Glu Gly Ser Gly Leu Gln Pro Lys Cys Pro Asp Thr Leu Gln Ser Arg
    690                 695                 700

Glu Gly Leu Gly Arg Met Glu Ser Phe Leu Thr Leu Glu Ser Glu Lys
705                 710                 715                 720

Ser Asp Phe Pro Pro Thr Pro Val Ala Glu Val Ala Pro Lys Ala Gln
                725                 730                 735

Glu Gly Glu Ser Thr Leu Glu Ile Arg Lys Met Gly Ser Cys Asp Gly
            740                 745                 750

Glu Gly Leu Leu Thr Ser Pro Asp Gln Pro Arg Gly Pro Ala Cys Asp
        755                 760                 765

Ala Ser Arg Gln Glu Phe His Ala Gly Val Pro His Pro Pro Gln Gly
    770                 775                 780

Glu Asn Leu Ala Ala Asp Leu Gly Leu Thr Ala Leu Ile Leu Asp Gln
785                 790                 795                 800

Asp Gln Gln Gly Ile Pro Ser Cys Pro Gly Glu Gly Trp Ile Arg Gly
                805                 810                 815

Ala Ala Ser Glu Trp Pro Leu Leu Ser Ser Glu Lys His Leu Gln Pro

            820                 825                 830

Ser Gln Ala Gln Pro Glu Thr Ser Ile Phe Asp Val Leu Lys Glu Gln
        835                 840                 845

Ala Gln Pro Pro Glu Asn Gly Lys Glu Thr Ser Pro Ser His Pro Gly
    850                 855                 860

Phe Lys Asp Gln Gly Ala Asp Ser Ser Gln Ile His Val Pro Val Glu
865                 870                 875                 880

Pro Gln Glu Asp Asn Asn Leu Pro Thr His Gly Gly Gln Glu Gln Ala
                885                 890                 895

Leu Gly Ser Glu Leu Gln Ser Gln Leu Pro Lys Gly Thr Leu Ser Asp
            900                 905                 910

Thr Pro Thr Ser Ser Pro Thr Asp Met Val Trp Glu Ser Ser Leu Thr
        915                 920                 925

Glu Glu Ser Glu Leu Ser Ala Pro Thr Arg Gln Lys Leu Pro Ala Leu
    930                 935                 940

Gly Glu Lys Arg Pro Glu Gly Ala Cys Gly Asp Gly Gln Ser Ser Arg
945                 950                 955                 960

Val Ser Pro Pro Ala Ala Asp Val Leu Lys Asp Phe Ser Leu Ala Gly
                965                 970                 975

Asn Phe Ser Arg Lys Glu Thr Cys Cys Thr Gly Gln Gly Pro Asn Lys
            980                 985                 990

Ser Gln Gln Ala Leu Ala Asp Ala Leu Glu Glu Gly Ser Gln His Glu
        995                 1000                1005

Glu Ala Cys Gln Arg His Pro Gly Ala Ser Glu Ala Ala Asp Gly
    1010                1015                1020

Cys Ser Pro Leu Trp Gly Leu Ser Lys Arg Glu Met Ala Ser Gly
    1025                1030                1035

Asn Thr Gly Glu Ala Pro Pro Cys Gln Pro Asp Ser Val Ala Leu
    1040                1045                1050

Leu Asp Ala Val Pro Cys Leu Pro Ala Leu Ala Pro Ala Ser Pro
    1055                1060                1065

Gly Val Thr Pro Thr Gln Asp Ala Pro Glu Thr Glu Ala Cys Asp
    1070                1075                1080

Glu Thr Gln Glu Gly Arg Gln Gln Pro Val Pro Ala Pro Gln Gln
    1085                1090                1095

Lys Met Glu Cys Trp Ala Thr Ser Asp Ala Glu Ser Pro Lys Leu
    1100                1105                1110

Leu Ala Ser Phe Pro Ser Ala Gly Glu Gln Gly Gly Glu Ala Gly
    1115                1120                1125

Ala Ala Glu Thr Gly Gly Ser Ala Gly Ala Gly Asp Pro Gly Lys
    1130                1135                1140

Gln Gln Ala Pro Glu Lys Pro Gly Glu Ala Thr Leu Ser Cys Gly
    1145                1150                1155

Leu Leu Gln Thr Glu His Cys Leu Thr Ser Gly Glu Glu Ala Ser
    1160                1165                1170

Thr Ser Ala Leu Arg Glu Ser Cys Gln Ala Glu His Pro Met Ala
    1175                1180                1185

Ser Cys Gln Asp Ala Leu Leu Pro Ala Arg Glu Leu Gly Gly Ile
    1190                1195                1200

Pro Arg Ser Thr Met Asp Phe Ser Thr His Gln Ala Val Pro Asp
    1205                1210                1215

Pro Lys Glu Leu Leu Leu Ser Gly Pro Pro Glu Val Ala Ala Pro
    1220                1225                1230

Asp Thr Pro Tyr Leu His Val Asp Ser Ala Ala Gln Arg Gly Ala
    1235                1240                1245

Glu Asp Ser Gly Val Lys Ala Val Ser Ser Ala Asp Pro Arg Ala
    1250                1255                1260

Pro Gly Glu Ser Pro Cys Pro Val Gly Glu Pro Pro Leu Ala Leu
    1265                1270                1275

Glu Asn Ala Ala Ser Leu Lys Leu Phe Ala Gly Ser Leu Ala Pro
    1280                1285                1290

Leu Leu Gln Pro Gly Ala Ala Gly Gly Glu Ile Pro Ala Val Gln
    1295                1300                1305

Ala Ser Ser Gly Ser Pro Lys Ala Arg Thr Thr Glu Gly Pro Val
    1310                1315                1320

Asp Ser Met Pro Cys Leu Asp Arg Met Pro Leu Leu Ala Lys Gly
    1325                1330                1335

Lys Gln Ala Thr Gly Glu Glu Lys Ala Ala Thr Ala Pro Gly Ala
    1340                1345                1350

Gly Ala Lys Ala Ser Gly Glu Gly Met Ala Gly Asp Ala Ala Gly
    1355                1360                1365

Glu Thr Glu Gly Ser Met Glu Arg Met Gly Glu Pro Ser Gln Asp
    1370                1375                1380

Pro Lys Gln Gly Thr Ser Gly Gly Val Asp Thr Ser Ser Glu Gln
    1385                1390                1395

Ile Ala Thr Leu Thr Gly Phe Pro Asp Phe Arg Glu His Ile Ala
    1400                1405                1410

Lys Ile Phe Glu Lys Pro Val Leu Gly Ala Leu Ala Thr Pro Gly
    1415                1420                1425

Glu Lys Ala Gly Ala Gly Arg Ser Ala Val Gly Lys Asp Leu Thr
    1430                1435                1440

Arg Pro Leu Gly Pro Glu Lys Leu Leu Asp Gly Pro Pro Gly Val
    1445                1450                1455

Asp Val Thr Leu Leu Pro Ala Pro Pro Ala Arg Leu Gln Val Glu
    1460                1465                1470

Lys Lys Gln Gln Leu Ala Gly Glu Ala Glu Ile Ser His Leu Ala
    1475                1480                1485

Leu Gln Asp Pro Ala Ser Asp Lys Leu Leu Gly Pro Ala Gly Leu
    1490                1495                1500

Thr Trp Glu Arg Asn Leu Pro Gly Ala Gly Val Gly Lys Glu Met
    1505                1510                1515

Ala Gly Val Pro Pro Thr Leu Arg Glu Asp Glu Arg Pro Glu Gly
    1520                1525                1530

Pro Gly Ala Ala Trp Pro Gly Leu Glu Gly Gln Ala Tyr Ser Gln
    1535                1540                1545

Leu Glu Arg Ser Arg Gln Glu Leu Ala Ser Gly Leu Pro Ser Pro
    1550                1555                1560

Ala Ala Thr Gln Glu Leu Pro Val Glu Arg Ala Ala Ala Phe Gln
    1565                1570                1575

Val Ala Pro His Ser His Gly Glu Glu Ala Val Ala Gln Asp Arg
    1580                1585                1590

Ile Pro Ser Gly Lys Gln His Gln Glu Thr Ser Ala Cys Asp Ser
    1595                1600                1605

Pro His Gly Glu Asp Gly Pro Gly Asp Phe Ala His Thr Gly Val
    1610                1615                1620

Pro Gly His Val Pro Arg Ser Thr Cys Ala Pro Ser Pro Gln Arg
    1625                1630                1635

Glu Val Leu Thr Val Pro Glu Ala Asn Ser Glu Pro Trp Thr Leu
    1640                1645                1650

Asp Thr Leu Gly Gly Glu Arg Arg Pro Gly Val Thr Ala Gly Ile
    1655                1660                1665

Leu Glu Met Arg Asn Ala Leu Gly Asn Gln Ser Thr Pro Ala Pro
    1670                1675                1680

Pro Thr Gly Glu Val Ala Asp Thr Pro Leu Glu Pro Gly Lys Val
    1685                1690                1695

Ala Gly Ala Ala Gly Glu Ala Glu Gly Asp Ile Thr Leu Ser Thr
    1700                1705                1710

Ala Glu Thr Gln Ala Cys Ala Ser Gly Asp Leu Pro Glu Ala Gly
    1715                1720                1725

Thr Thr Arg Thr Phe Ser Val Val Ala Gly Asp Leu Val Leu Pro
    1730                1735                1740

Gly Ser Cys Gln Asp Pro Ala Cys Ser Asp Lys Ala Pro Gly Met
    1745                1750                1755

Glu Gly Thr Ala Ala Leu His Gly Asp Ser Pro Ala Arg Pro Gln
    1760                1765                1770

Gln Ala Lys Glu Gln Pro Gly Pro Glu Arg Pro Ile Pro Ala Gly

1775                1780                1785

Asp Gly Lys Val Cys Val Ser Ser Pro Pro Glu Pro Asp Glu Thr
    1790                1795                1800

His Asp Pro Lys Leu Gln His Leu Ala Pro Glu Glu Leu His Thr
    1805                1810                1815

Asp Arg Glu Ser Pro Arg Pro Gly Pro Ser Met Leu Pro Ser Val
    1820                1825                1830

Pro Lys Lys Asp Ala Pro Arg Val Met Asp Lys Val Thr Ser Asp
    1835                1840                1845

Glu Thr Arg Gly Ala Glu Gly Thr Glu Arg Ser Ala Lys Asp Ile
    1850                1855                1860

Gly Leu Trp Lys Ala Met Met Pro Ser Leu Asp Thr Asp Thr Leu
    1865                1870                1875

Asp Val Leu Glu Arg Arg Val Val Leu Ile Gln His Glu Phe Leu
    1880                1885                1890

His Lys Lys Glu Ala Asn Gly His Ser Arg Phe Met Tyr Ser Ser
    1895                1900                1905

Pro Val Ala Asp Asp Ile Ile Gln Pro Ala Ala Pro Ala Asp Leu
    1910                1915                1920

Glu Ser Pro Thr Leu Ala Ala Ser Ser Tyr His Gly Asp Val Val
    1925                1930                1935

Gly Gln Val Ser Thr Asp Leu Ile Ala Gln Arg Ser Ser Asp Ser
    1940                1945                1950

Glu Glu Ala Phe Glu Thr Pro Glu Ser Thr Thr Pro Val Lys Ala
    1955                1960                1965

Pro Pro Ala Pro Pro Pro Pro Pro Pro Glu Val Ile Pro Glu Pro
    1970                1975                1980

Glu Val Ser Thr Gln Pro Pro Pro Glu Glu Pro Gly Cys Gly Ser
    1985                1990                1995

Glu Thr Val Pro Val Pro Asp Gly Pro Arg Ser Asp Ser Val Glu
    2000                2005                2010

Gly Ser Pro Phe Arg Pro Pro Ser His Ser Phe Ser Ala Val Phe
    2015                2020                2025

Asp Glu Asp Lys Pro Ile Ala Ser Ser Gly Thr Tyr Asn Leu Asp
    2030                2035                2040

Phe Asp Asn Ile Glu Leu Val Asp Thr Phe Gln Thr Leu Glu Pro
    2045                2050                2055

Arg Ala Ser Asp Ala Lys Asn Gln Glu Gly Lys Val Asn Thr Arg
    2060                2065                2070

Arg Lys Ser Thr Asp Ser Val Pro Ile Ser Lys Ser Thr Leu Ser
    2075                2080                2085

Arg Ser Leu Ser Leu Gln Ala Ser Asp Phe Asp Gly Ala Ser Ser
    2090                2095                2100

Ser Gly Asn Pro Glu Ala Val Ala Leu Ala Pro Asp Ala Tyr Ser
    2105                2110                2115

Thr Gly Ser Ser Ser Ala Ser Ser Thr Leu Lys Arg Thr Lys Lys
    2120                2125                2130

Pro Arg Pro Pro Ser Leu Lys Lys Lys Gln Thr Thr Lys Lys Pro
    2135                2140                2145

Thr Glu Thr Pro Pro Val Lys Glu Thr Gln Gln Glu Pro Asp Glu
    2150                2155                2160

Glu Ser Leu Val Pro Ser Gly Glu Asn Leu Ala Ser Glu Thr Lys
    2165                2170                2175

Thr Glu Ser Ala Lys Thr Glu Gly Pro Ser Pro Ala Leu Leu Glu
    2180                2185                2190

Glu Thr Pro Leu Glu Pro Ala Val Gly Pro Lys Ala Ala Cys Pro
    2195                2200                2205

Leu Asp Ser Glu Ser Ala Glu Gly Val Val Pro Pro Ala Ser Gly
    2210                2215                2220

Gly Gly Arg Val Gln Asn Ser Pro Pro Val Gly Arg Lys Thr Leu
    2225                2230                2235

Pro Leu Thr Thr Ala Pro Glu Ala Gly Glu Val Thr Pro Ser Asp
    2240                2245                2250

Ser Gly Gly Gln Glu Asp Ser Pro Ala Lys Gly Leu Ser Val Arg
    2255                2260                2265

Leu Glu Phe Asp Tyr Ser Glu Asp Lys Ser Ser Trp Asp Asn Gln
    2270                2275                2280

Gln Glu Asn Pro Pro Pro Thr Lys Lys Ile Gly Lys Lys Pro Val
    2285                2290                2295

Ala Lys Met Pro Leu Arg Arg Pro Lys Met Lys Lys Thr Pro Glu
    2300                2305                2310

Lys Leu Asp Asn Thr Pro Ala Ser Pro Pro Arg Ser Pro Ala Glu
    2315                2320                2325

Pro Asn Asp Ile Pro Ile Ala Lys Gly Thr Tyr Thr Phe Asp Ile
    2330                2335                2340

Asp Lys Trp Asp Asp Pro Asn Phe Asn Pro Phe Ser Ser Thr Ser
    2345                2350                2355

Lys Met Gln Glu Ser Pro Lys Leu Pro Gln Gln Ser Tyr Asn Phe
    2360                2365                2370

Asp Pro Asp Thr Cys Asp Glu Ser Val Asp Pro Phe Lys Thr Ser
    2375                2380                2385

Ser Lys Thr Pro Ser Ser Pro Ser Lys Ser Pro Ala Ser Phe Glu
    2390                2395                2400

Ile Pro Ala Ser Ala Met Glu Ala Asn Gly Val Asp Gly Asp Gly
    2405                2410                2415

Leu Asn Lys Pro Ala Lys Lys Lys Lys Thr Pro Leu Lys Thr Asp
    2420                2425                2430

Thr Phe Arg Val Lys Lys Ser Pro Lys Arg Ser Pro Leu Ser Asp
    2435                2440                2445

Pro Pro Ser Gln Asp Pro Thr Pro Ala Ala Thr Pro Glu Thr Pro
    2450                2455                2460

Pro Val Ile Ser Ala Val Val His Ala Thr Asp Glu Glu Lys Leu
    2465                2470                2475

Ala Val Thr Asn Gln Lys Trp Thr Cys Met Thr Val Asp Leu Glu
    2480                2485                2490

Ala Asp Lys Gln Asp Tyr Pro Gln Pro Ser Asp Leu Ser Thr Phe
    2495                2500                2505

Val Asn Glu Thr Lys Phe Ser Ser Pro Thr Glu Glu Leu Asp Tyr
    2510                2515                2520

Arg Asn Ser Tyr Glu Ile Glu Tyr Met Glu Lys Ile Gly Ser Ser
    2525                2530                2535

Leu Pro Gln Asp Asp Asp Ala Pro Lys Lys Gln Ala Leu Tyr Leu
    2540                2545                2550

Met Phe Asp Thr Ser Gln Glu Ser Pro Val Lys Ser Ser Pro Val
    2555                2560                2565

Arg Met Ser Glu Ser Pro Thr Pro Cys Ser Gly Ser Ser Phe Glu
    2570                2575                2580

Glu Thr Glu Ala Leu Val Asn Thr Ala Ala Lys Asn Gln His Pro
    2585                2590                2595

Val Pro Arg Gly Leu Ala Pro Asn Gln Glu Ser His Leu Gln Val
    2600                2605                2610

Pro Glu Lys Ser Ser Gln Lys Glu Leu Glu Ala Met Gly Leu Gly
    2615                2620                2625

Thr Pro Ser Glu Ala Ile Glu Ile Arg Glu Ala Ala His Pro Thr
    2630                2635                2640

Asp Val Ser Ile Ser Lys Thr Ala Leu Tyr Ser Arg Ile Gly Thr
    2645                2650                2655

Ala Glu Val Glu Lys Pro Ala Gly Leu Leu Phe Gln Gln Pro Asp
    2660                2665                2670

Leu Asp Ser Ala Leu Gln Ile Ala Arg Ala Glu Ile Ile Thr Lys
    2675                2680                2685

Glu Arg Glu Val Ser Glu Trp Lys Asp Lys Tyr Glu Glu Ser Arg
    2690                2695                2700

Arg Glu Val Met Glu Met Arg Lys Ile Val Ala Glu Tyr Glu Lys
    2705                2710                2715

Thr Ile Ala Gln Met Ile Glu Asp Glu Gln Arg Glu Lys Ser Val

2720                    2725                    2730

Ser His Gln Thr Val Gln Gln Leu Val Leu Glu Lys Glu Gln Ala
    2735                    2740                    2745

Leu Ala Asp Leu Asn Ser Val Glu Lys Ser Leu Ala Asp Leu Phe
    2750                    2755                    2760

Arg Arg Tyr Glu Lys Met Lys Glu Val Leu Glu Gly Phe Arg Lys
    2765                    2770                    2775

Asn Glu Glu Val Leu Lys Arg Cys Ala Gln Glu Tyr Leu Ser Arg
    2780                    2785                    2790

Val Lys Lys Glu Glu Gln Arg Tyr Gln Ala Leu Lys Val His Ala
    2795                    2800                    2805

Glu Glu Lys Leu Asp Arg Ala Asn Ala Glu Ile Ala Gln Val Arg
    2810                    2815                    2820

Gly Lys Ala Gln Gln Glu Gln Ala Ala His Gln Ala Ser Leu Arg
    2825                    2830                    2835

Lys Glu Gln Leu Arg Val Asp Ala Leu Glu Arg Thr Leu Glu Gln
    2840                    2845                    2850

Lys Asn Lys Glu Ile Glu Glu Leu Thr Lys Ile Cys Asp Glu Leu
    2855                    2860                    2865

Ile Ala Lys Met Gly Lys Ser
    2870                    2875

<210>  7
<211>  2826
<212>  PRT
<213>  Homo sapiens

<400>  7

Met Gly Asn Glu Asn Ser Thr Ser Asp Asn Gln Arg Thr Leu Ser Ala
1               5                   10                  15

Gln Thr Pro Arg Ser Ala Gln Pro Pro Gly Asn Ser Gln Asn Ile Lys
            20                  25                  30

Arg Lys Gln Gln Asp Thr Pro Gly Ser Pro Asp His Arg Asp Ala Ser
        35                  40                  45

Ser Ile Gly Ser Val Gly Leu Gly Gly Phe Cys Thr Ala Ser Glu Ser

50                  55                  60

Ser Ala Ser Leu Asp Pro Cys Leu Val Ser Pro Glu Val Thr Glu Pro
65                  70                  75                  80

Arg Lys Asp Pro Gln Gly Ala Arg Gly Pro Glu Gly Ser Leu Leu Pro
                85                  90                  95

Ser Pro Pro Pro Ser Gln Glu Arg Glu His Pro Ser Ser Ser Met Pro
            100                 105                 110

Phe Ala Glu Cys Pro Pro Glu Gly Cys Leu Ala Ser Pro Ala Ala Ala
        115                 120                 125

Pro Glu Asp Gly Pro Gln Thr Gln Ser Pro Arg Arg Glu Pro Ala Pro
    130                 135                 140

Asn Ala Pro Gly Asp Ile Ala Ala Ala Phe Pro Ala Glu Arg Asp Ser
145                 150                 155                 160

Ser Thr Pro Tyr Gln Glu Ile Ala Ala Val Pro Ser Ala Gly Arg Glu
                165                 170                 175

Arg Gln Pro Lys Glu Glu Gly Gln Lys Ser Ser Phe Ser Phe Ser Ser
            180                 185                 190

Gly Ile Asp Gln Ser Pro Gly Met Ser Pro Val Pro Leu Arg Glu Pro
        195                 200                 205

Met Lys Ala Pro Leu Cys Gly Glu Gly Asp Gln Pro Gly Gly Phe Glu
    210                 215                 220

Ser Gln Glu Lys Glu Ala Ala Gly Gly Phe Pro Pro Ala Glu Ser Arg
225                 230                 235                 240

Gln Gly Val Ala Ser Val Gln Val Thr Pro Glu Ala Pro Ala Ala Ala
                245                 250                 255

Gln Gln Gly Thr Glu Ser Ser Ala Val Leu Glu Lys Ser Pro Leu Lys
            260                 265                 270

Pro Met Ala Pro Ile Pro Gln Asp Pro Ala Pro Arg Ala Ser Asp Arg
        275                 280                 285

Glu Arg Gly Gln Gly Glu Ala Pro Pro Gln Tyr Leu Thr Asp Asp Leu
    290                 295                 300

Glu Phe Leu Arg Ala Cys His Leu Pro Arg Ser Asn Ser Gly Ala Ala
305                 310                 315                 320

Pro Glu Ala Glu Val Asn Ala Ala Ser Gln Glu Ser Cys Gln Gln Pro
                325                 330                 335

Val Gly Ala Tyr Leu Pro His Ala Glu Leu Pro Trp Gly Leu Pro Ser
            340                 345                 350

Pro Ala Leu Val Pro Glu Ala Gly Gly Ser Gly Lys Glu Ala Leu Asp
        355                 360                 365

Thr Ile Asp Val Gln Gly His Pro Gln Thr Gly Met Arg Gly Thr Lys
    370                 375                 380

Pro Asn Gln Val Val Cys Val Ala Ala Gly Gly Gln Pro Glu Gly Gly
385                 390                 395                 400

Leu Pro Val Ser Pro Glu Pro Ser Leu Leu Thr Pro Thr Glu Glu Ala
                405                 410                 415

His Pro Ala Ser Ser Leu Ala Ser Phe Pro Ala Ala Gln Ile Pro Ile
            420                 425                 430

Ala Val Glu Glu Pro Gly Ser Ser Ser Arg Glu Ser Val Ser Lys Ala
        435                 440                 445

Gly Met Pro Val Ser Ala Asp Ala Ala Lys Glu Val Val Asp Ala Gly
    450                 455                 460

Leu Val Gly Leu Glu Arg Gln Val Ser Asp Leu Gly Ser Lys Gly Glu
465                 470                 475                 480

His Pro Glu Gly Asp Pro Gly Glu Val Pro Ala Pro Ser Pro Gln Glu
                485                 490                 495

Arg Gly Glu His Leu Asn Thr Glu Gln Ser His Glu Val Gln Pro Gly
            500                 505                 510

Val Pro Pro Pro Pro Leu Pro Lys Glu Gln Ser His Glu Val Gln Pro
        515                 520                 525

Gly Ala Pro Pro Pro Pro Leu Pro Lys Ala Pro Ser Glu Ser Ala Arg
    530                 535                 540

Gly Pro Pro Gly Pro Thr Asp Gly Ala Lys Val His Glu Asp Ser Thr
545                 550                 555                 560

Ser Pro Ala Val Ala Lys Glu Gly Ser Arg Ser Pro Gly Asp Ser Pro
                565                 570                 575

Gly Gly Lys Glu Glu Ala Pro Glu Pro Pro Asp Gly Gly Asp Pro Gly
            580                 585                 590

Asn Leu Gln Gly Glu Asp Ser Gln Ala Phe Ser Ser Lys Arg Asp Pro
        595                 600                 605

Glu Val Gly Lys Asp Glu Leu Ser Lys Pro Ser Ser Asp Ala Glu Ser
    610                 615                 620

Arg Asp His Pro Ser Ser His Ser Ala Gln Pro Pro Arg Lys Gly Gly
625                 630                 635                 640

Ala Gly His Thr Asp Gly Pro His Ser Gln Thr Ala Glu Ala Asp Ala
                645                 650                 655

Ser Gly Leu Pro His Lys Leu Gly Glu Glu Asp Pro Val Leu Pro Pro
            660                 665                 670

Val Pro Asp Gly Ala Gly Glu Pro Thr Val Pro Glu Gly Ala Ile Trp
        675                 680                 685

Glu Gly Ser Gly Leu Gln Pro Lys Cys Pro Asp Thr Leu Gln Ser Arg
    690                 695                 700

Glu Gly Leu Gly Arg Met Glu Ser Phe Leu Thr Leu Glu Ser Glu Lys
705                 710                 715                 720

Ser Asp Phe Pro Pro Thr Pro Val Ala Glu Val Ala Pro Lys Ala Gln
                725                 730                 735

Glu Gly Glu Ser Thr Leu Glu Ile Arg Lys Met Gly Ser Cys Asp Gly
            740                 745                 750

Glu Gly Leu Leu Thr Ser Pro Asp Gln Pro Arg Gly Pro Ala Cys Asp
        755                 760                 765

Ala Ser Arg Gln Glu Phe His Ala Gly Val Pro His Pro Pro Gln Gly
    770                 775                 780

Glu Asn Leu Ala Ala Asp Leu Gly Leu Thr Ala Leu Ile Leu Asp Gln
785                 790                 795                 800

Asp Gln Gln Gly Ile Pro Ser Cys Pro Gly Glu Gly Trp Ile Arg Gly
                805                 810                 815

Ala Ala Ser Glu Trp Pro Leu Leu Ser Ser Glu Lys His Leu Gln Pro
            820                 825                 830

Ser Gln Ala Gln Pro Glu Thr Ser Ile Phe Asp Val Leu Lys Glu Gln
        835                 840                 845

Ala Gln Pro Pro Glu Asn Gly Lys Glu Thr Ser Pro Ser His Pro Gly
    850                 855                 860

Phe Lys Asp Gln Gly Ala Asp Ser Ser Gln Ile His Val Pro Val Glu
865                 870                 875                 880

Pro Gln Glu Asp Asn Asn Leu Pro Thr His Gly Gly Gln Glu Gln Ala
                885                 890                 895

Leu Gly Ser Glu Leu Gln Ser Gln Leu Pro Lys Gly Thr Leu Ser Asp
            900                 905                 910

Thr Pro Thr Ser Ser Pro Thr Asp Met Val Trp Glu Ser Ser Leu Thr
        915                 920                 925

Glu Glu Ser Glu Leu Ser Ala Pro Thr Arg Gln Lys Leu Pro Ala Leu
    930                 935                 940

Gly Glu Lys Arg Pro Glu Gly Ala Cys Gly Asp Gly Gln Ser Ser Arg
945                 950                 955                 960

Val Ser Pro Pro Ala Ala Asp Val Leu Lys Asp Phe Ser Leu Ala Gly
                965                 970                 975

Asn Phe Ser Arg Lys Glu Thr Cys Cys Thr Gly Gln Gly Pro Asn Lys
            980                 985                 990

Ser Gln Gln Ala Leu Ala Asp Ala  Leu Glu Glu Gly Ser  Gln His Glu
        995                 1000                1005

Glu Ala  Cys Gln Arg His Pro  Gly Ala Ser Glu Ala  Ala Asp Gly
    1010                1015                1020

Cys Ser  Pro Leu Trp Gly Leu  Ser Lys Arg Glu Met  Ala Ser Gly
    1025                1030                1035

Asn Thr  Gly Glu Ala Pro Pro  Cys Gln Pro Asp Ser  Val Ala Leu
    1040                1045                1050

Leu Asp  Ala Val Pro Cys Leu  Pro Ala Leu Ala Pro  Ala Ser Pro

1055                1060                1065

Gly Val  Thr Pro Thr Gln Asp  Ala Pro Glu Thr Glu  Ala Cys Asp
    1070                1075                1080

Glu Thr  Gln Glu Gly Arg Gln  Gln Pro Val Pro Ala  Pro Gln Gln
    1085                1090                1095

Lys Met  Glu Cys Trp Ala Thr  Ser Asp Ala Glu Ser  Pro Lys Leu
    1100                1105                1110

Leu Ala  Ser Phe Pro Ser Ala  Gly Glu Gln Gly Gly  Glu Ala Gly
    1115                1120                1125

Ala Ala  Glu Thr Gly Gly Ser  Ala Gly Ala Gly Asp  Pro Gly Lys
    1130                1135                1140

Gln Gln  Ala Pro Glu Lys Pro  Gly Glu Ala Thr Leu  Ser Cys Gly
    1145                1150                1155

Leu Leu  Gln Thr Glu His Cys  Leu Thr Ser Gly Glu  Glu Ala Ser
    1160                1165                1170

Thr Ser  Ala Leu Arg Glu Ser  Cys Gln Ala Glu His  Pro Met Ala
    1175                1180                1185

Ser Cys  Gln Asp Ala Leu Leu  Pro Ala Arg Glu Leu  Gly Gly Ile
    1190                1195                1200

Pro Arg  Ser Thr Met Asp Phe  Ser Thr His Gln Ala  Val Pro Asp
    1205                1210                1215

Pro Lys  Glu Leu Leu Leu Ser  Gly Pro Pro Glu Val  Ala Ala Pro
    1220                1225                1230

Asp Thr  Pro Tyr Leu His Val  Asp Ser Ala Ala Gln  Arg Gly Ala
    1235                1240                1245

Glu Asp  Ser Gly Val Lys Ala  Val Ser Ser Ala Asp  Pro Arg Ala
    1250                1255                1260

Pro Gly  Glu Ser Pro Cys Pro  Val Gly Glu Pro Pro  Leu Ala Leu
    1265                1270                1275

Glu Asn  Ala Ala Ser Leu Lys  Leu Phe Ala Gly Ser  Leu Ala Pro
    1280                1285                1290

Leu Leu Gln Pro Gly Ala Ala Gly Gly Glu Ile Pro Ala Val Gln
    1295                1300                1305

Ala Ser Ser Gly Ser Pro Lys Ala Arg Thr Thr Glu Gly Pro Val
    1310                1315                1320

Asp Ser Met Pro Cys Leu Asp Arg Met Pro Leu Leu Ala Lys Gly
    1325                1330                1335

Lys Gln Ala Thr Gly Glu Glu Lys Ala Ala Thr Ala Pro Gly Ala
    1340                1345                1350

Gly Ala Lys Ala Ser Gly Glu Gly Met Ala Gly Asp Ala Ala Gly
    1355                1360                1365

Glu Thr Glu Gly Ser Met Glu Arg Met Gly Glu Pro Ser Gln Asp
    1370                1375                1380

Pro Lys Gln Gly Thr Ser Gly Gly Val Asp Thr Ser Ser Glu Gln
    1385                1390                1395

Ile Ala Thr Leu Thr Gly Phe Pro Asp Phe Arg Glu His Ile Ala
    1400                1405                1410

Lys Ile Phe Glu Lys Pro Val Leu Gly Ala Leu Ala Thr Pro Gly
    1415                1420                1425

Glu Lys Ala Gly Ala Gly Arg Ser Ala Val Gly Lys Asp Leu Thr
    1430                1435                1440

Arg Pro Leu Gly Pro Glu Lys Leu Leu Asp Gly Pro Pro Gly Val
    1445                1450                1455

Asp Val Thr Leu Leu Pro Ala Pro Pro Ala Arg Leu Gln Val Glu
    1460                1465                1470

Lys Lys Gln Gln Leu Ala Gly Glu Ala Glu Ile Ser His Leu Ala
    1475                1480                1485

Leu Gln Asp Pro Ala Ser Asp Lys Leu Leu Gly Pro Ala Gly Leu
    1490                1495                1500

Thr Trp Glu Arg Asn Leu Pro Gly Ala Gly Val Gly Lys Glu Met
    1505                1510                1515

Ala Gly Val Pro Pro Thr Leu Arg Glu Asp Glu Arg Pro Glu Gly
    1520                1525                1530

Pro Gly Ala Ala Trp Pro Gly Leu Glu Gly Gln Ala Tyr Ser Gln
    1535                1540                1545

Leu Glu Arg Ser Arg Gln Glu Leu Ala Ser Gly Leu Pro Ser Pro
    1550                1555                1560

Ala Ala Thr Gln Glu Leu Pro Val Glu Arg Ala Ala Ala Phe Gln
    1565                1570                1575

Val Ala Pro His Ser His Gly Glu Glu Ala Val Ala Gln Asp Arg
    1580                1585                1590

Ile Pro Ser Gly Lys Gln His Gln Glu Thr Ser Ala Cys Asp Ser
    1595                1600                1605

Pro His Gly Glu Asp Gly Pro Gly Asp Phe Ala His Thr Gly Val
    1610                1615                1620

Pro Gly His Val Pro Arg Ser Thr Cys Ala Pro Ser Pro Gln Arg
    1625                1630                1635

Glu Val Leu Thr Val Pro Glu Ala Asn Ser Glu Pro Trp Thr Leu
    1640                1645                1650

Asp Thr Leu Gly Gly Glu Arg Arg Pro Gly Val Thr Ala Gly Ile
    1655                1660                1665

Leu Glu Met Arg Asn Ala Leu Gly Asn Gln Ser Thr Pro Ala Pro
    1670                1675                1680

Pro Thr Gly Glu Val Ala Asp Thr Pro Leu Glu Pro Gly Lys Val
    1685                1690                1695

Ala Gly Ala Ala Gly Glu Ala Glu Gly Asp Ile Thr Leu Ser Thr
    1700                1705                1710

Ala Glu Thr Gln Ala Cys Ala Ser Gly Asp Leu Pro Glu Ala Gly
    1715                1720                1725

Thr Thr Arg Thr Phe Ser Val Val Ala Gly Asp Leu Val Leu Pro
    1730                1735                1740

Gly Ser Cys Gln Asp Pro Ala Cys Ser Asp Lys Ala Pro Gly Met
    1745                1750                1755

Glu Gly Thr Ala Ala Leu His Gly Asp Ser Pro Ala Arg Pro Gln
    1760                1765                1770

Gln Ala Lys Glu Gln Pro Gly Pro Glu Arg Pro Ile Pro Ala Gly
    1775                1780                1785

Asp Gly Lys Val Cys Val Ser Ser Pro Pro Glu Pro Asp Glu Thr
    1790                1795                1800

His Asp Pro Lys Leu Gln His Leu Ala Pro Glu Glu Leu His Thr
    1805                1810                1815

Asp Arg Glu Ser Pro Arg Pro Gly Pro Ser Met Leu Pro Ser Val
    1820                1825                1830

Pro Lys Lys Asp Ala Pro Arg Val Met Asp Lys Val Thr Ser Asp
    1835                1840                1845

Glu Thr Arg Gly Ala Glu Gly Thr Glu Ser Ser Pro Val Ala Asp
    1850                1855                1860

Asp Ile Ile Gln Pro Ala Ala Pro Ala Asp Leu Glu Ser Pro Thr
    1865                1870                1875

Leu Ala Ala Ser Ser Tyr His Gly Asp Val Val Gly Gln Val Ser
    1880                1885                1890

Thr Asp Leu Ile Ala Gln Arg Ser Ser Asp Ser Glu Glu Ala Phe
    1895                1900                1905

Glu Thr Pro Glu Ser Thr Thr Pro Val Lys Ala Pro Pro Ala Pro
    1910                1915                1920

Pro Pro Pro Pro Pro Glu Val Ile Pro Glu Pro Glu Val Ser Thr
    1925                1930                1935

Gln Pro Pro Pro Glu Glu Pro Gly Cys Gly Ser Glu Thr Val Pro
    1940                1945                1950

Val Pro Asp Gly Pro Arg Ser Asp Ser Val Glu Gly Ser Pro Phe
    1955                1960                1965

Arg Pro Pro Ser His Ser Phe Ser Ala Val Phe Asp Glu Asp Lys
    1970                1975                1980

Pro Ile Ala Ser Ser Gly Thr Tyr Asn Leu Asp Phe Asp Asn Ile
    1985                1990                1995

Glu Leu Val Asp Thr Phe Gln Thr Leu Glu Pro Arg Ala Ser Asp

2000                2005                2010

Ala Lys Asn Gln Glu Gly Lys Val Asn Thr Arg Arg Lys Ser Thr
    2015                2020                2025

Asp Ser Val Pro Ile Ser Lys Ser Thr Leu Ser Arg Ser Leu Ser
    2030                2035                2040

Leu Gln Ala Ser Asp Phe Asp Gly Ala Ser Ser Ser Gly Asn Pro
    2045                2050                2055

Glu Ala Val Ala Leu Ala Pro Asp Ala Tyr Ser Thr Gly Ser Ser
    2060                2065                2070

Ser Ala Ser Ser Thr Leu Lys Arg Thr Lys Lys Pro Arg Pro Pro
    2075                2080                2085

Ser Leu Lys Lys Lys Gln Thr Thr Lys Lys Pro Thr Glu Thr Pro
    2090                2095                2100

Pro Val Lys Glu Thr Gln Gln Glu Pro Asp Glu Glu Ser Leu Val
    2105                2110                2115

Pro Ser Gly Glu Asn Leu Ala Ser Glu Thr Lys Thr Glu Ser Ala
    2120                2125                2130

Lys Thr Glu Gly Pro Ser Pro Ala Leu Leu Glu Glu Thr Pro Leu
    2135                2140                2145

Glu Pro Ala Val Gly Pro Lys Ala Ala Cys Pro Leu Asp Ser Glu
    2150                2155                2160

Ser Ala Glu Gly Val Val Pro Pro Ala Ser Gly Gly Gly Arg Val
    2165                2170                2175

Gln Asn Ser Pro Pro Val Gly Arg Lys Thr Leu Pro Leu Thr Thr
    2180                2185                2190

Ala Pro Glu Ala Gly Glu Val Thr Pro Ser Asp Ser Gly Gly Gln
    2195                2200                2205

Glu Asp Ser Pro Ala Lys Gly Leu Ser Val Arg Leu Glu Phe Asp
    2210                2215                2220

Tyr Ser Glu Asp Lys Ser Ser Trp Asp Asn Gln Gln Glu Asn Pro
    2225                2230                2235

Pro Pro Thr Lys Lys Ile Gly Lys Lys Pro Val Ala Lys Met Pro
    2240                2245                2250

Leu Arg Arg Pro Lys Met Lys Lys Thr Pro Glu Lys Leu Asp Asn
    2255                2260                2265

Thr Pro Ala Ser Pro Pro Arg Ser Pro Ala Glu Pro Asn Asp Ile
    2270                2275                2280

Pro Ile Ala Lys Gly Thr Tyr Thr Phe Asp Ile Asp Lys Trp Asp
    2285                2290                2295

Asp Pro Asn Phe Asn Pro Phe Ser Ser Thr Ser Lys Met Gln Glu
    2300                2305                2310

Ser Pro Lys Leu Pro Gln Gln Ser Tyr Asn Phe Asp Pro Asp Thr
    2315                2320                2325

Cys Asp Glu Ser Val Asp Pro Phe Lys Thr Ser Ser Lys Thr Pro
    2330                2335                2340

Ser Ser Pro Ser Lys Ser Pro Ala Ser Phe Glu Ile Pro Ala Ser
    2345                2350                2355

Ala Met Glu Ala Asn Gly Val Asp Gly Asp Gly Leu Asn Lys Pro
    2360                2365                2370

Ala Lys Lys Lys Lys Thr Pro Leu Lys Thr Asp Thr Phe Arg Val
    2375                2380                2385

Lys Lys Ser Pro Lys Arg Ser Pro Leu Ser Asp Pro Pro Ser Gln
    2390                2395                2400

Asp Pro Thr Pro Ala Ala Thr Pro Glu Thr Pro Pro Val Ile Ser
    2405                2410                2415

Ala Val Val His Ala Thr Asp Glu Glu Lys Leu Ala Val Thr Asn
    2420                2425                2430

Gln Lys Trp Thr Cys Met Thr Val Asp Leu Glu Ala Asp Lys Gln
    2435                2440                2445

Asp Tyr Pro Gln Pro Ser Asp Leu Ser Thr Phe Val Asn Glu Thr
    2450                2455                2460

Lys Phe Ser Ser Pro Thr Glu Glu Leu Asp Tyr Arg Asn Ser Tyr
    2465                2470                2475

Glu Ile Glu Tyr Met Glu Lys Ile Gly Ser Ser Leu Pro Gln Asp
    2480                2485                2490

Asp Asp Ala Pro Lys Lys Gln Ala Leu Tyr Leu Met Phe Asp Thr
    2495                2500                2505

Ser Gln Glu Ser Pro Val Lys Ser Ser Pro Val Arg Met Ser Glu
    2510                2515                2520

Ser Pro Thr Pro Cys Ser Gly Ser Ser Phe Glu Glu Thr Glu Ala
    2525                2530                2535

Leu Val Asn Thr Ala Ala Lys Asn Gln His Pro Val Pro Arg Gly
    2540                2545                2550

Leu Ala Pro Asn Gln Glu Ser His Leu Gln Val Pro Glu Lys Ser
    2555                2560                2565

Ser Gln Lys Glu Leu Glu Ala Met Gly Leu Gly Thr Pro Ser Glu
    2570                2575                2580

Ala Ile Glu Ile Arg Glu Ala Ala His Pro Thr Asp Val Ser Ile
    2585                2590                2595

Ser Lys Thr Ala Leu Tyr Ser Arg Ile Gly Thr Ala Glu Val Glu
    2600                2605                2610

Lys Pro Ala Gly Leu Leu Phe Gln Gln Pro Asp Leu Asp Ser Ala
    2615                2620                2625

Leu Gln Ile Ala Arg Ala Glu Ile Ile Thr Lys Glu Arg Glu Val
    2630                2635                2640

Ser Glu Trp Lys Asp Lys Tyr Glu Glu Ser Arg Arg Glu Val Met
    2645                2650                2655

Glu Met Arg Lys Ile Val Ala Glu Tyr Glu Lys Thr Ile Ala Gln
    2660                2665                2670

Met Ile Glu Asp Glu Gln Arg Glu Lys Ser Val Ser His Gln Thr
    2675                2680                2685

Val Gln Gln Leu Val Leu Glu Lys Glu Gln Ala Leu Ala Asp Leu
    2690                2695                2700

Asn Ser Val Glu Lys Ser Leu Ala Asp Leu Phe Arg Arg Tyr Glu
    2705                2710                2715

Lys Met Lys Glu Val Leu Glu Gly Phe Arg Lys Asn Glu Glu Val
    2720                2725                2730

Leu Lys Arg Cys Ala Gln Glu Tyr Leu Ser Arg Val Lys Lys Glu
    2735                2740                2745

Glu Gln Arg Tyr Gln Ala Leu Lys Val His Ala Glu Glu Lys Leu
    2750                2755                2760

Asp Arg Ala Asn Ala Glu Ile Ala Gln Val Arg Gly Lys Ala Gln
    2765                2770                2775

Gln Glu Gln Ala Ala His Gln Ala Ser Leu Arg Lys Glu Gln Leu
    2780                2785                2790

Arg Val Asp Ala Leu Glu Arg Thr Leu Glu Gln Lys Asn Lys Glu
    2795                2800                2805

Ile Glu Glu Leu Thr Lys Ile Cys Asp Glu Leu Ile Ala Lys Met
    2810                2815                2820

Gly Lys Ser
    2825

<210>  8
<211>  20
<212>  DNA
<213>  Artificial Sequence

<220>
<223>  Synthetic oligonucleotide

<400>  8
ccagttgctg aagggcagaa                                                20

<210>  9
<211>  19
<212>  DNA
<213>  Artificial Sequence

<220>
<223>  Synthetic oligonucleotide

<400>  9
gcggaccttg gagtctgag                                                 19

<210>  10
<211>  20
<212>  DNA
<213>  Artificial Sequence

<220>
<223>  Synthetic oligonucleotide

<400>  10
caaagtgcca gtgactggaa                                                   20


<210>  11
<211>  20
<212>  DNA
<213>  Artificial Sequence

<220>
<223>  Synthetic oligonucleotide

<400>  11
cacattctgt attggcatct                                                   20


<210>  12
<211>  20
<212>  DNA
<213>  Artificial Sequence

<220>
<223>  Synthetic oligonucleotide

<400>  12
tctgccagac tccaaattat                                                   20


<210>  13
<211>  20
<212>  DNA
<213>  Artificial Sequence

<220>
<223>  Synthetic oligonucleotide

<400>  13
gatgtagtgg tggtcatgag                                                   20


<210>  14
<211>  20
<212>  DNA
<213>  Artificial Sequence

<220>
<223>  Synthetic oligonucleotide

<400>  14
gccttaagaa aagtcgtcca                                                   20


<210>  15
<211>  20
<212>  DNA
<213>  Artificial Sequence

<220>
<223>  Synthetic oligonucleotide

<400>  15
tcataggttt tgcttggaca                                                   20


<210>  16
<211>  20
<212>  DNA
<213>  Artificial Sequence

<220>
<223>  Synthetic oligonucleotide

<400>  16
cagagtgccg ctttatgaaa                                                   20


<210>  17
<211>  20
<212>  DNA
<213>  Artificial Sequence

<220>
<223>  Synthetic oligonucleotide

<400>  17
gtacaattgt tgctgcttca                                                   20


<210>  18
<211>  20
<212>  DNA
<213>  Artificial Sequence

<220>
<223>  Synthetic oligonucleotide

<400>  18
tctctggcaa gacaacagtc                                                   20


<210>  19
<211>  20
<212>  DNA
<213>  Artificial Sequence

<220>
<223>  Synthetic oligonucleotide

<400>  19
gtcgccatat tttacatctt                                                   20


<210>  20
<211>  20
<212>  DNA
<213>  Artificial Sequence

<220>
<223>  Synthetic oligonucleotide

<400>  20
gcttgttggg atttaggtat                                                   20

<210>  21
<211>  20
<212>  DNA
<213>  Artificial Sequence

<220>
<223>  Synthetic oligonucleotide

<400>  21
gggctttgca gtaatacatc                                                    20


<210>  22
<211>  20
<212>  DNA
<213>  Artificial Sequence

<220>
<223>  Synthetic oligonucleotide

<400>  22
ttgtatctca accgtgactc                                                    20


<210>  23
<211>  20
<212>  DNA
<213>  Artificial Sequence

<220>
<223>  Synthetic oligonucleotide

<400>  23
agattattgt cttgtgcctt                                                    20


<210>  24
<211>  20
<212>  DNA
<213>  Artificial Sequence

<220>
<223>  Synthetic oligonucleotide

<400>  24
   taaaccccct atttttagga                                                    20


<210>  25
<211>  20
<212>  DNA
<213>  Artificial Sequence

<220>
<223>  Synthetic oligonucleotide

<400>  25
tgcatggtaa attccatgct                                                    20


<210>  26
<211>  20

<212>  DNA
<213>  Artificial Sequence

<220>
<223>  Synthetic oligonucleotide

<400>  26
tttgtgtctg acctcttatt                                                   20


<210>  27
<211>  20
<212>  DNA
<213>  Artificial Sequence

<220>
<223>  Synthetic oligonucleotide

<400>  27
gctacttata ccctagtgaa                                                   20


<210>  28
<211>  20
<212>  DNA
<213>  Artificial Sequence

<220>
<223>  Synthetic oligonucleotide

<400>  28
ccatgcatat tcagagggaa                                                   20


<210>  29
<211>  20
<212>  DNA
<213>  Artificial Sequence

<220>
<223>  Synthetic oligonucleotide

<400>  29
gccacagatc ttgcattaag                                                   20


<210>  30
<211>  20
<212>  DNA
<213>  Artificial Sequence

<220>
<223>  Synthetic oligonucleotide

<400>  30
taattgcttt gagctcgcta                                                   20


<210>  31
<211>  20
<212>  DNA
<213>  Artificial Sequence

<220>
<223>  Synthetic oligonucleotide

<400>  31
gggattctat ttagcagcat                                                   20


<210>  32
<211>  20
<212>  DNA
<213>  Artificial Sequence

<220>
<223>  Synthetic oligonucleotide

<400>  32
attcttccac tactgttgtg                                                   20


<210>  33
<211>  20
<212>  DNA
<213>  Artificial Sequence

<220>
<223>  Synthetic oligonucleotide

<400>  33
agggcatctg acacattttg                                                   20


<210>  34
<211>  20
<212>  DNA
<213>  Artificial Sequence

<220>
<223>  Synthetic oligonucleotide

<400>  34
tgctaaccac tcacaatacc                                                   20

## Description

### Field of the Invention

### (Related Application and Incorporation by Reference)

The present application claims the priority of Japanese Patent Application No. 2015-138104 filed on July 9, 2015, the whole contents of which are incorporated herein by reference.

All literatures cited herein are incorporated herein by reference in their entirety for every purpose. The citation of any literature is not to be construed as an admission that it is prior art with respect to the present invention.

The present invention relates to an approach for discriminating at a molecular level between endometrial cancer with lymph node metastasis and endometrial cancer without metastasis.

### Background of the Invention

Endometrial cancer is a cancer reportedly having good prognosis with a recurrence rate of approximately 10% and a 5-year survival rate exceeding 90% in early cancer at stage I. The Gynecologic Oncology Group (GOG) study in 1180 patients in stages I and II shows that postoperative survival rates are good when they do not have risk factors such as cervical stromal invasion, positive peritoneal cytology, adnexal metastasis, and lymph node metastasis.

Surgical therapy is the first-line choice of the standard initial treatment of early endometrial cancer. However, for endometrial cancer, it is difficult to test beforehand the presence or absence of lymph node metastasis, which is a risk factor and serves as a criterion for confirming stages. Therefore, in addition to total hysterectomy and adnexectomy, lymph node dissection is carried out as standard operative procedures, and the stage is decided by the pathological diagnosis of the harvested lymph nodes.

The 5-year survival rate of such early endometrial cancer is generally good and approximately 85% to 95%. However, not only are there the problems of side effects of lymph node dissection, such as intractable lymphedema, but the pathological diagnosis depends on the morphological judgment of individual diagnosticians. In addition, criteria for making definitive diagnosis have latent problems in themselves, such as a risk of overlooking micrometastasis based on its principle. In fact, several % of patients experience recurrence within 5 years, in spite of having undergone lymph node dissection. Thus, the development of novel methods for recurrence prevention or treatment is demanded for such patients having poor prognosis.

Meanwhile, molecular marker development has also been actively performed as to endometrial cancer, and core protein-related molecules, including CA125, in blood are exploited as biomarkers in diagnosis. CA125 is a marker whose detection value and positivity rate are elevated as the stage progresses, and can be exploited in the assessment of not only cancer aggravation but therapeutic effects. However, this marker cannot be exploited as a diagnostic criterion for initiating recurrence prevention for early cancer patients having a small tumor mass, due to low positivity rates in those patients.

Thus, if a risk of lymph node metastasis can be confirmed by assaying specimens obtained by preoperative primary lesion curettage or immediately assaying surgically harvested primary cancer lesions, lymph node dissection can be avoided for patients having a low risk of metastasis. Hence, the patients can rid themselves of their fear for intractable complications. Furthermore, the achievement of effective individualized medicine greatly advantageous for patients can be expected.

Semaphorins are a group of proteins which were identified as guidance molecules involved in cell-cell signaling, and are involved in the formation of blood vessels or neural circuits or the regulation of immunocytes. The semaphorins are divided into 7 classes (subfamilies) according to difference in the sequence of a part adjacent to the Sema domain. Class 3 semaphorins have been found to function as important regulatory factors for cell processes such as survival, growth, apoptosis, and transition of vascular endothelial cells and tumor cells, and to regulate the morphology or migration of cells. Among them, class 3D semaphorin (SEMA3D) has been reported to inhibit breast cancer development or tumor formation and blood vessel formation attributed thereto (Non Patent Literature 1).

However, the relationship between SEMA3D and endometrial cancer or the relationship between SEMA3D and lymph node metastasis in endometrial cancer has heretofore been totally unknown.

### Citation List

### Non Patent Literature

Non Patent Literature 1: Kigel B, Varshavsky A, Kessler O, Neufeld G. Successful inhibition of tumor development by specific class-3 semaphorins is associated with expression of appropriate semaphorin receptors by tumor cells. PLoS ONE 2008;3: e3287

### Summary of the Invention

### Problems to be Solved by the Invention

The present invention relates to the identification of a marker molecule which differs in expression between endometrial cancer with lymph node metastasis and endometrial cancer without metastasis, and the provision of an approach for molecularly discriminating endometrial cancer between endometrial cancer with lymph node metastasis and endometrial cancer without metastasis by the measurement of the marker.

### Means for Solving the Problems

The present inventors have measured and analyzed the expression at a nucleic acid level of SEMA3D expressed in cancer tissues of endometrial cancer patients. As a result, the present inventors have found that the expression level of SEMA3D is a significantly higher value in a lymph node metastasis-negative group compared with a lymph node metastasis-positive group, and found that SEMA3D is useful as a marker for discriminating between endometrial cancer with lymph node metastasis and endometrial cancer without metastasis. The present inventors have also found that combined use of the SEMA3D with TACC2 permits more accurate discrimination between endometrial cancer with lymph node metastasis and endometrial cancer without metastasis.

Specifically, the present invention relates to the following 1) to 13):
1) A method for assessing lymph node metastasis or lymph node metastatic potential, or prognosis of endometrial cancer, comprising the step of measuring an expression level of SEMA3D in a biological sample derived from a cancer tissue isolated from an endometrial cancer patient.
2) The method according to 1), wherein the method comprises the following steps a and b:
   a) the step of measuring an expression level of SEMA3D in a biological sample derived from a cancer tissue isolated from an endometrial cancer patient; and
   b) the step of comparing the expression level of SEMA3D with a control level or a threshold level.
3) The method according to 1) or 2), wherein when the expression level of SEMA3D is larger than the control level or the threshold level, it is determined that there is no lymph node metastasis, lymph node metastatic potential is low, or prognosis is good.
4) The method according to any of 1) to 3), wherein the expression level of SEMA3D in the biological sample is an amount of a transcription product or a translation product of SEMA3D in an endometrial cancer tissue.
5) A method for assessing lymph node metastasis or lymph node metastatic potential, or prognosis of endometrial cancer, comprising the step of measuring expression levels of SEMA3D and TACC2 in a biological sample derived from a cancer tissue isolated from an endometrial cancer patient.
6) The method according to 5), wherein the method comprises the following steps c and d:
   c) the step of measuring expression levels of SEMA3D and TACC2 in a biological sample derived from a cancer tissue isolated from an endometrial cancer patient; and
   d) the step of comparing the expression levels of SEMA3D and TACC2 with a control level or a threshold level.
7) The method according to 5) or 6), wherein when a relative expression level of the expression level of SEMA3D compared with the expression level of TACC2 is larger than the control level or the threshold level, it is determined that there is no lymph node metastasis, lymph node metastatic potential is low, or prognosis is good.
8) The method according to any of 5) to 7), wherein the expression levels of SEMA3D and TACC2 in the biological sample are amounts of transcription products or translation products of SEMA3D and TACC2 in an endometrial cancer tissue.
9) A testing kit for assessing lymph node metastasis or lymph node metastatic potential, or prognosis of endometrial cancer for use in the method according to any of 1) to 4), the testing kit comprising an antibody specifically binding to SEMA3D protein, or an oligonucleotide specifically recognizing a nucleic acid derived from SEMA3D gene.
10) A testing kit for assessing lymph node metastasis or lymph node metastatic potential, or prognosis of endometrial cancer for use in the method according to any of 5) to 8), the testing kit comprising an antibody specifically binding to SEMA3D protein and an antibody specifically binding to TACC2 protein, or an oligonucleotide specifically recognizing a nucleic acid derived from SEMA3D gene and an oligonucleotide specifically recognizing a nucleic acid derived from TACC2 gene.
11) An apparatus for assessing lymph node metastasis or lymph node metastatic potential, or prognosis of endometrial cancer for use in the method according to any of 1) to 8).
12) Use of SEMA3D as a marker for assessing lymph node metastasis or lymph node metastatic potential, or prognosis of endometrial cancer.
13) Use of SEMA3D and TACC2 as markers for assessing lymph node metastasis or lymph node metastatic potential, or prognosis of endometrial cancer.

### Effects of the Invention

According to the present invention, an endometrial cancer specimen having no chance to metastasize to the lymph nodes can be selected. This suggests a policy regarding operative procedures, also including the avoidance of unnecessary lymph node dissection. Provided that the lymph node dissection can be avoided, shortening of operative duration, mitigation of operative stress, and prevention of postoperative lymphedema or the like can be achieved. Also, use of the present invention allows lymph node metastasis or lymph node metastatic potential, or prognosis of endometrial cancer to be objectively determined at a level equivalent to or higher than the subjectivity of specialists such as well-trained clinical laboratory technicians. Thus, the present invention can also be suitably used in point of care testing (POCT) from the collection of specimens from patients to the analysis thereof.

### Brief Description of the Drawings

[Figure 1] Figure 1 shows the relative RNA expression level of SEMA3D in a lymph node metastasis-positive group and -negative group.
[Figure 2] Figure 2 shows a ROC curve of the relative RNA expression level of SEMA3D. Ordinate: sensitivity, abscissa: false positivity rate (= 1-specificity).
[Figure 3] Figure 3 shows the relative RNA expression level of TACC2 (TACC2 isoform L or S) in a lymph node metastasis-positive group and -negative group.
[Figure 4] Figure 4 shows the relative RNA expression level of SEMA3D to the RNA expression level of TACC2 (TACC2 isoform L or S) in a lymph node metastasis-positive group and -negative group.
[Figure 5] Figure 5 is a scatter diagram of the relative RNA expression levels of SEMA3D and TACC2 (TACC2 isoform L or S).
[Figure 6] Figure 6 shows a ROC curve of the relative RNA expression level of a combination (SEMA3D-TACC2) of SEMA3D and TACC2 (TACC2 isoform L or S). Ordinate: sensitivity, abscissa: false positivity rate (= 1-specificity).
[Figure 7] Figure 7A shows a hematoxylin-eosin staining image of endometrial cancer cells. Figure 7B shows a fluorescent *in situ* hybridization (FISH) image of SEMA3D mRNA. The SEMA3D mRNA is shown in green, and nucleus stained with DAPI is shown in blue. Figure 7C shows a negative control for Figure 7B. Nucleus stained with DAPI is shown in blue. Figure 7D shows an immunostaining image of SEMA3D protein. Figure 7E shows a negative control for Figure 7D. Figure 7F shows an immunostaining image of TACC2 protein. Figure 7G shows a negative control for Figure 7F.

### Modes for Carrying out the Invention

In the present invention, the endometrial cancer means a cancer which develops in the uterine corpus among uterine cancers. The endometrial cancer arises from the endometrium, which is an epithelial tissue lining the cavity of the uterus, and is also called uterine body cancer or uterine corpus cancer, etc. The stages of the endometrial cancer are classified into I to IV according to the Japan Society of Obstetrics and Gynecology (JSOG) classification of clinical stages (2011) in view of the tumor size, spread, and invasion or metastasis statuses of the cancer. In the present invention, the endometrial cancer to be assessed is not particularly limited by its stage and is preferably well-differentiated adenocarcinoma of endometrioid adenocarcinoma.

In the present invention, the lymph node metastasis of the endometrial cancer means that the endometrial cancer grows in, for example, the intrapelvic or para-aortic lymph nodes.

In the present invention, the assessment of lymph node metastasis means that the endometrial cancer is evaluated or assayed for the presence or absence of its lymph node metastasis. The assessment of lymph node metastatic potential (lymph node metastatic property) means that the endometrial cancer is evaluated or assayed for whether or not to have the ability to metastasize to the lymph nodes and grow therein.

In the present invention, the assessment of prognosis means that the course and end of endometrial cancer are predicted. Good or poor prognosis of endometrial cancer is closely related to metastasis of the cancer, and a principal pathway in the metastasis of cancer cells is lymph nodes. Thus, the assessment of lymph node metastatic potential closely correlates with the assessment of prognosis of endometrial cancer. The prognosis can be assessed as being poor when the lymph node metastasis (metastatic potential) is present, and can be assessed as being good when the lymph node metastasis (metastatic potential) is absent.

The biological sample used in the present invention is an endometrial cancer tissue isolated before or during operation from an endometrial cancer patient to be assessed. The biological sample is appropriately prepared and/or treated for assay. For example, in the case of assaying the sample at a nucleic acid level, RNA extracts are prepared. In the case of assaying the sample at a protein level, protein extracts are prepared.

Any method known in the art can be used as a method for extracting RNA from the biological sample. Specific examples thereof can include Ambion RiboPure kit (manufactured by Life Technologies Corp.), miRNeasy (manufactured by Qiagen N.V.), and RNeasy (manufactured by Qiagen N.V.). Of them, miRNeasy kit manufactured by Qiagen N.V. is preferably used.

In the present specification, the term "nucleic acid" means DNA or RNA. The "DNA" encompasses not only double-stranded DNA but each single-stranded DNA as a sense strand and an antisense strand constituting the double-stranded DNA. Thus, the DNA encompasses, for example, double-stranded genomic DNA, single-stranded cDNA, and single-stranded DNA having a sequence complementary to the DNA. The "RNA" includes all of total RNA, mRNA, rRNA, and synthetic RNA.

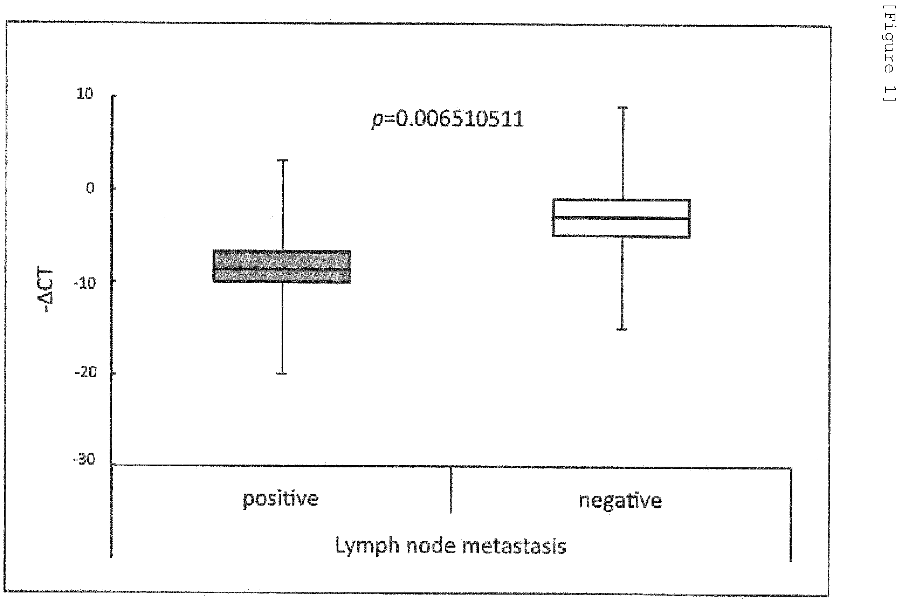

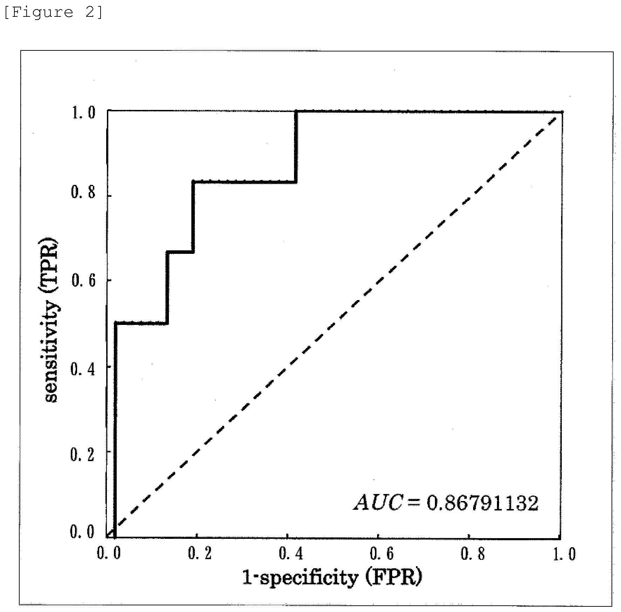

As shown later in Examples, the expression level of SEMA3D was measured in patient with endometrial adenocarcinoma G1 and G2 localized to the uterine corpus (lymph node metastasis-positive: 7 patients, lymph node metastasis-negative: 53 patients), and was consequently a significantly higher value in the lymph node metastasis-negative group compared with the lymph node metastasis-positive group (Figure 1). Both sensitivity and specificity were shown to be higher in the negative group (Figure 2). Thus, SEMA3D can serve as a marker for assessing lymph node metastasis or lymph node metastatic potential, or prognosis of endometrial cancer.

SEMA3D gene and protein are registered in NCBI as follows:
SEMA3D gene: NM_152754 (SEQ ID NO: 1)
SEMA3D protein: NP_689967 (SEQ ID NO: 2)

More highly accurate assessment can be performed by measuring the expression level of TACC2 in combination with the expression level of SEMA3D.

TACC2 is transforming acidic coiled-coil-containing protein 2 (UniProtKB/Swiss-Prot: TACC2#HUMAN (O95359)). In the present invention, a TACC2 isoform residing in the gene locus of TACC2 can also be used. In this context, examples of the TACC2 isoform include a gene comprising a nucleotide sequence represented by SEQ ID NO: 3 (TACC2 isoform L) or 4 (TACC2 isoform S) having a site from start position 123779316 to termination position 123779341 at chromosome number 10 (chr10) of human reference genome (GRCh37) as a transcription start site (TSS), and any or a portion of proteins encoded thereby, i.e., UniProtKB/Swiss-Prot: TACC2#HUMAN (O95359, SEQ ID NO: 5), E9PBC6#HUMAN (E9PBC6, SEQ ID NO: 6), and E7EMZ9#HUMAN (E7EMZ9, SEQ ID NO: 7). The present applicants have already found that the TACC2 isoform can serve as a marker for assessing lymph node metastasis of endometrial cancer (PCT/JP2015/050551).

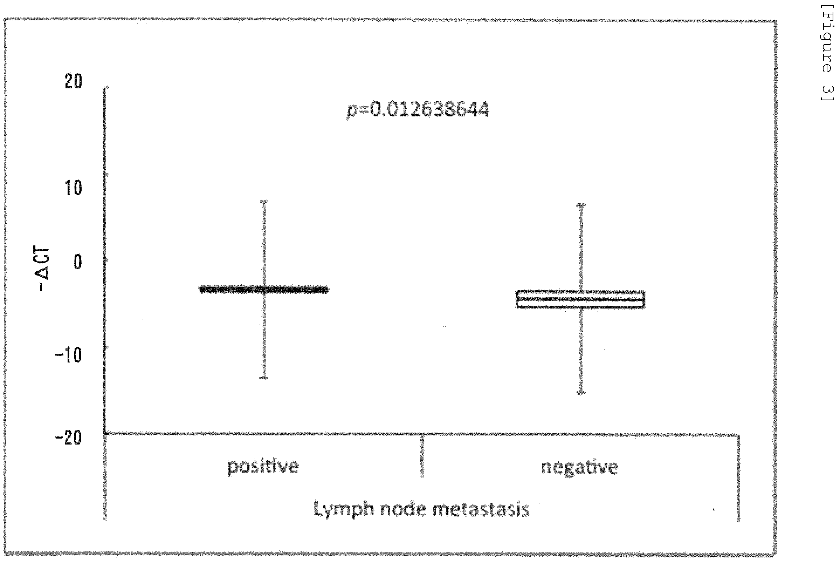

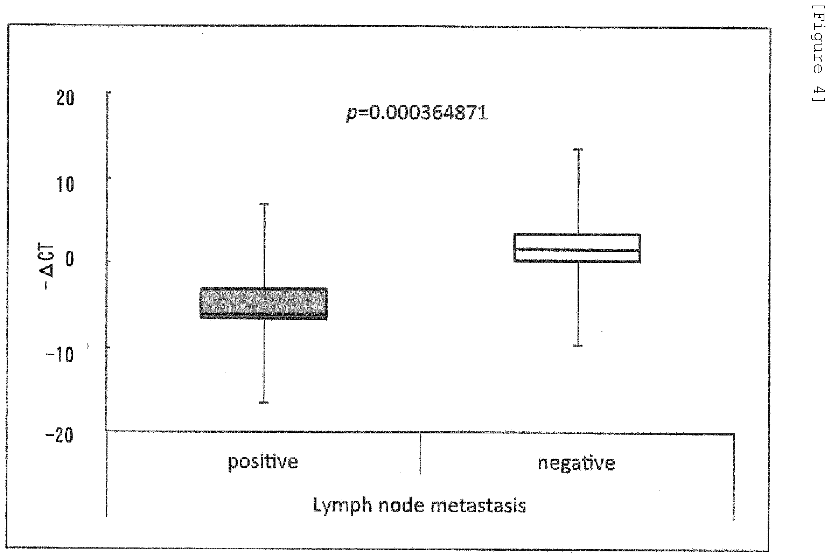

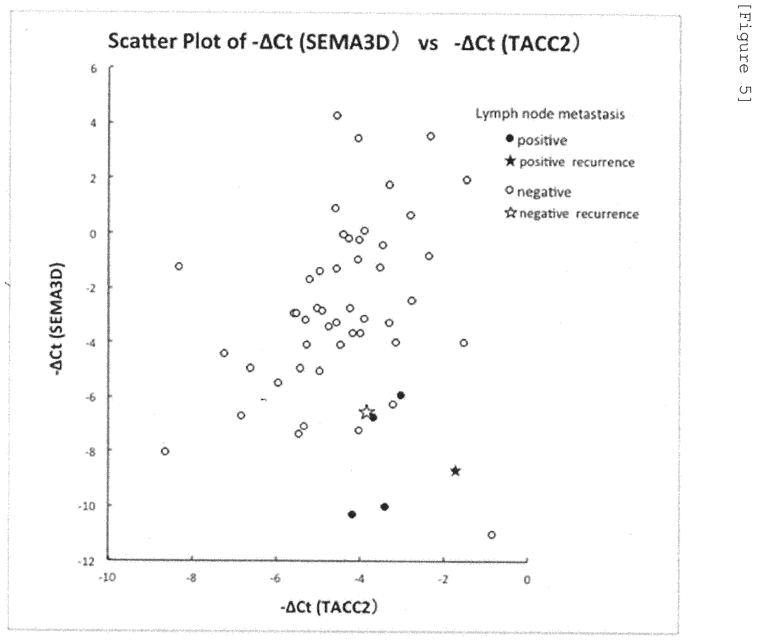

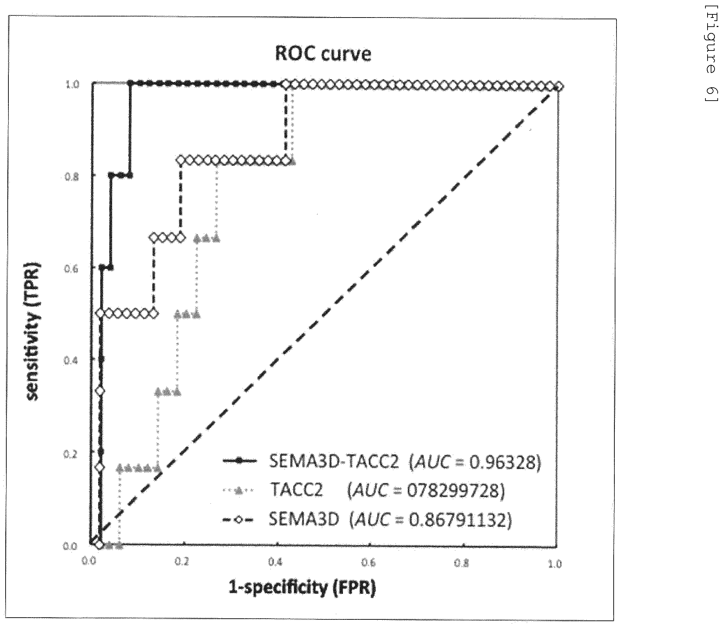

As shown later in Examples, the gene expression level of TACC2 is a significantly higher value in a lymph node metastasis-positive group compared with a lymph node metastasis-negative group (Figure 3), and the relative expression level of the SEMA3D and TACC2 genes in combination, -ΔCt (SEMA3D-TACC2), indicates low expression in the lymph node metastasis-positive group and high expression in the negative group (Figure 4). Also, the lymph node metastasis-positive group has a low value of -ΔCt (SEMA3D) and a high value of -ΔCt (TACC2). Thus, the expression levels of SEMA3D and TACC2 were confirmed to negatively correlate with each other in diagnosis of lymph node metastasis (Figure 5). Furthermore, the combined use of SEMA3D and TACC2 is superior in sensitivity and specificity to the use of each marker alone (Figure 6).

Thus, the combination of SEMA3D and TACC2 is very useful as a marker for assessing lymph node metastasis or lymph node metastatic potential, or prognosis of endometrial cancer.

In the present invention, the method for measuring the expression level of SEMA3D, or SEMA3D and TACC2 (hereinafter, these are collectively referred to as a "marker") is not particularly limited as long as the method is capable of confirming the marker in a biological sample. Specifically, any method which can detect or quantify the expression level of the marker can be used. Preferred examples thereof include detection or quantification of the marker at a nucleic acid level, specifically, detection or quantification of a transcription product (mRNA), and detection or quantification at a protein level, specifically, detection or quantification of a translation product (protein).

The detection or quantification method at a nucleic acid level can be selected from nucleic acid amplification methods typified by PCR using DNA primers hybridizing to a nucleic acid derived from the marker gene, RT-PCR, SmartAmp, and LAMP, hybridization methods (DNA chips, DNA microarrays, dot blot hybridization, slot blot hybridization, Northern blot hybridization, etc.) using nucleic acid probes hybridizing to the nucleic acid derived from the marker gene, sequencing methods, and combinations of these methods.

In this context, the probe or the primer for use in the assay corresponds to a primer for specifically recognizing and amplifying the nucleic acid derived from the marker gene, or a probe for specifically detecting the nucleic acid derived from the marker gene. These can be designed on the basis of the nucleotide sequence represented by SEQ ID NO: 1, 3, or 4. In this context, the phrase "specifically recognizing" means that substantially only the nucleic acid derived from the marker gene can be detected, for example, in Northern blot, and substantially the detected matter or the product can be determined as the nucleic acid derived from the marker gene, for example, in RT-PCR, in such a way that substantially only the nucleic acid is formed.

Specifically, an oligonucleotide comprising a given number of nucleotides complementary to the DNA comprising any of the nucleotide sequences represented by SEQ ID NOs: 1 and 3 to 4 or a complementary strand thereof can be used. In this context, the "complementary strand" refers to another strand against one strand of double-stranded DNA composed of A:T (U for RNA) and G:C base pairs. The term "complementary" is not limited by a completely complementary sequence in a region with the given number of consecutive nucleotides and needs only to have preferably 80% or higher, more preferably 90% or higher, even more preferably 95% or higher nucleotide sequence identity. The identity of the nucleotide sequence can be determined using, for example, a homology calculation algorithm NCBI BLAST under conditions involving expected value = 10, gap accepted, filtering = ON, match score = 1, and mismatch score = -3.

For use as a primer, such an oligonucleotide is not particularly limited as long as the oligonucleotide is capable of specific annealing and strand elongation. Examples thereof include oligonucleotides usually having a length of, for example, 10 or more bases, preferably 15 or more bases, more preferably 20 or more bases, and, for example, 100 or less bases, preferably 50 or less bases, more preferably 35 or less bases.

For use as a probe, the oligonucleotide is not particularly limited as long as the oligonucleotide is capable of specific hybridization. An oligonucleotide having at least a portion or the whole sequence of the DNA comprising any of the nucleotide sequences represented by SEQ ID NOs: 1 and 3 to 4 (or a complementary strand thereof) and having a length of, for example, 10 or more bases, preferably 15 or more bases, and, for example, 100 or less bases, preferably 50 or less bases, more preferably 25 or less bases is used.

The specific hybridization means hybridization substantially only to the nucleic acid of interest. Examples thereof include hybridization under stringent conditions. In this context, examples of the stringent conditions can typically include washing conditions on the order of "1 × SSC, 0.1% SDS, 37°C" and can include more stringent hybridization conditions on the order of "0.5 × SSC, 0.1% SDS, 42°C" and even more stringent hybridization conditions on the order of "0.1 × SSC, 0.1% SDS, 65°C". The hybridization conditions are described in, for example, J. Sambrook et al., Molecular Cloning: A Laboratory Manual, Third Edition, Cold Spring Harbor Laboratory Press (2001).

The "oligonucleotide" can be DNA or RNA and may be synthetic or natural. The probe for use in hybridization is usually labeled and then used. Examples of the labeling agent include radioisotopes (e.g., ³²P, ³³P, ³⁵S, ¹²⁵I, ¹³¹I, ³H, and ¹⁴C), enzymes (e.g., β-galactosidase, β-glucosidase, alkaline phosphatase, peroxidase, and malate dehydrogenase), fluorescent materials (e.g., fluorescamine and fluorescein isothiocyanate), and luminescent materials (e.g., luminol, luminol derivatives, luciferin, and lucigenin).

For example, in the case of utilizing Northern blot hybridization, first, probe DNA is labeled with a radioisotope, a fluorescent material, or the like, and the obtained labeled DNA is subsequently hybridized with biological sample-derived RNA transferred to a nylon membrane or the like according to a routine method. Then, the formed double strand of the labeled DNA and the RNA can be confirmed by use of a method for detecting and measuring a signal derived from the label.

In the case of utilizing RT-PCR, first, cDNA is prepared from biological sample-derived RNA according to a routine method. This cDNA is used as a template and hybridized with a pair of primers (a forward strand binding to the cDNA (- strand) and a reverse strand binding to the + strand) prepared so as to be capable of amplifying the target expression product (transcription product) of the marker. Then, PCR is performed according to a routine method, and the obtained amplified double-stranded DNA is detected. The detection of the amplified double-stranded DNA can employ, for example, a method which involves detecting labeled double-stranded DNA produced by PCR described above using primers labeled in advance with RI, a fluorescent material, or the like.

In the case of measuring the expression level of mRNA in a specimen using a DNA microarray, an array in which at least one nucleic acid (cDNA or DNA) derived from the marker gene is immobilized on a support is used. Labeled cDNA or cRNA prepared from the mRNA is allowed to bind onto the microarray. The mRNA expression level can be measured by detecting the label on the microarray.

The nucleic acid to be immobilized on the array can be a nucleic acid capable of hybridization under stringent conditions and may be, for example, a nucleic acid having the whole sequence of the marker gene or may be a nucleic acid consisting of a partial sequence thereof. In this context, examples of the "partial sequence" include a nucleic acid consisting of at least 15 to 25 bases.

In the case of comparing the differential expression of the marker in the assay described above, a measurement value such as an expression level can be corrected in advance as a relative value to that of a gene having a relatively stable expression level. This correction permits more accurate comparison of the differential expression of the marker among a plurality of independent biological samples. The correction of the measurement value can be performed by correcting the expression level of the marker gene with that of an internal standard gene whose expression level does not largely vary depending on the presence or absence of lymph node metastasis or lymph node metastatic potential of endometrial cancer. In this context, a housekeeping gene GAPDH is generally used as the gene having a stable expression level. In the present invention, SUDS3 gene (NM_022491) was confirmed to exhibit a more stable expression level in endometrial cancer cells and was used as an internal standard.

In the present invention, the measurement of the expression level of the marker also includes the measurement of the expression level of a homolog of the marker having biological activity equivalent to that of the marker.

Examples of such a homolog gene include a polynucleotide which comprises a nucleotide sequence hybridizing under stringent conditions to a complementary sequence of the nucleotide sequence represented by SEQ ID NO: 1, 3, or 4, and has biological activity equivalent to that of a protein consisting of any of the amino acid sequences represented by SEQ ID NOs: 2 and 5 to 7.

In this context, examples of the stringent conditions can include, as described above, "1 × SSC, 0.1% SDS, 37°C", more stringently "0.5 × SSC, 0.1% SDS, 42°C", even more stringently "0.1 × SSC, 0.1% SDS, 65°C". Examples of such a polynucleotide can specifically include a polynucleotide comprising a nucleotide sequence having 80% or higher, preferably 90% or higher, more preferably 95% or higher identity to the nucleotide sequence represented by SEQ ID NO: 1, 3, or 4. The identity of the nucleotide sequence can be determined by the BLAST algorithm or the like.

The detection or quantification of the marker at a protein level is performed conveniently and preferably by an immunological assay method using an antibody specifically binding to the marker protein (anti-SEMA3D antibody with or without an anti-TACC2 antibody). Examples of the immunological assay method include enzyme-linked immunosorbent assay (ELISA), radioimmunoassay, immunohistochemical staining, Western blot, immunoprecipitation, immunofluorescence, and flow cytometry.

According to, for example, Western blot, the antibody described above is used as a primary antibody. Then, for example, radioisotope-, fluorescent material- or enzyme-labeled antibody binding to the primary antibody is used as a secondary antibody to label the primary antibody. A signal derived from such a labeling material is measured using a radiation counter, a fluorescence detector, or the like.

The anti-SEMA3D antibody or the anti-TACC2 antibody may be a polyclonal antibody or may be a monoclonal antibody. Alternatively, a portion (partial fragment) of the antibody or a peptide comprising a portion of the antibody may be used as long as the antibody fragment maintains the binding activity of the antibody against the antigen (marker protein). Examples of such an antibody fragment include F(ab')₂, Fab', Fab, and single-chain Fv (scFv).

These antibodies can be produced according to methods known in the art. Specifically, the polyclonal antibody can be obtained according to a routine method from the serum of an immunized animal obtained by immunizing a nonhuman animal (e.g., a rabbit) with a protein expressed in *E. coli* or the like and purified according to a routine method or with a partial polypeptide of the protein synthesized according to a routine method.

On the other hand, the monoclonal antibody can be obtained from hybridoma cells prepared by immunizing a nonhuman animal (e.g., a mouse) with a protein expressed in *E. coli* or the like and purified according to a routine method or with a partial polypeptide of the protein and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

The antibody fragment can be obtained by the enzymatic treatment of the obtained antibody or by use of sequence information of the obtained antibody.

In the case of conducting an immunohistochemical analysis method, the biological sample isolated from a patient is fixed in formalin by a routine method, then embedded in paraffin, and sliced into a tissue section, which is attached to slide glass. The resultant is preferably used as a section sample. An antibody labeled with an enzyme such as alkaline phosphatase or peroxidase can be used as the secondary antibody. Highly sensitive detection is preferably performed using, for example, a three-step method such as ABC or LSAB, or DAKO EnVision detection system.

In this way, the expression level of the marker in the biological sample derived from a cancer tissue isolated from an endometrial cancer patient is measured. The presence or absence of lymph node metastasis or lymph node metastatic potential, or poor prognosis is assessed on the basis of the expression level. Specifically, the detected expression level of the marker is compared with a control level for the assessment.

In this context, examples of the "control level" include the expression level of the marker in an endometrial cancer tissue isolated from endometrial cancer patient without lymph node metastasis or in a normal tissue isolated from the endometrial cancer patient, and the expression level of the marker in a healthy individual group having no endometrial cancer.

For example, when the expression level of SEMA3D in the cancer tissue of the subject patient is close to the expression level in an endometrial cancer tissue isolated from an endometrial cancer patient without lymph node metastasis, a normal tissue, or a tissue derived from a healthy individual, when the expression level of SEMA3D in the cancer tissue of the subject patient belongs to within the range of this expression level, or when the expression level of SEMA3D in the cancer tissue of the subject patient is significantly higher than this expression level, the endometrial cancer of the patient can be assessed as having no lymph node metastasis, low lymph node metastatic potential, or good prognosis.

Alternatively, when the relative expression level of SEMA3D to the expression level of TACC2 in the cancer tissue of the subject patient is close to the expression level in an endometrial cancer tissue isolated from an endometrial cancer patient without lymph node metastasis, a normal tissue, or a tissue derived from a healthy individual, when the relative expression level of SEMA3D to the expression level of TACC2 in the cancer tissue of the subject patient belongs to within the range of this expression level, or when the relative expression level of SEMA3D to the expression level of TACC2 in the cancer tissue of the subject patient is significantly higher than this expression level, the endometrial cancer of the patient can be assessed as having no lymph node metastasis, low lymph node metastatic potential, or good prognosis.

The assessment of lymph node metastasis or lymph node metastatic potential of endometrial cancer according to the present invention can also be conducted on the basis of increase or decrease in the expression level of the marker. In this case, a reference value (threshold level) is established on the basis of the control level, for example, the expression level of the marker derived from a normal tissue, an endometrial cancer tissue isolated from an endometrial cancer patient without lymph node metastasis, or a tissue of a healthy individual.
The assessment can be conducted by comparing the expression level of the marker in the patient-derived biological sample with the reference value (e.g., a range of ± 2S.D. is used as a tolerance). For example, when the expression level of SEMA3D in the patient-derived biological sample is higher than the threshold level, the endometrial cancer of the patient can be assessed as having no lymph node metastasis, low lymph node metastatic potential, or good prognosis. Alternatively, when the relative expression level of SEMA3D to the expression level of TACC2 in the patient-derived biological sample is higher than the threshold level, the endometrial cancer of the patient can be assessed as having no lymph node metastasis, low lymph node metastatic potential, or good prognosis.

The possibility of lymph node metastasis or lymph node metastatic potential, or prognosis is determined on the basis of information provided according to the method of the present invention as described above, if necessary combined with an additional method (CT, MRI, PET-CT, etc.). Although criteria for determining whether to perform lymph node biopsy or lymph node dissection are left to physician's judgment, for example, lymph node dissection can be performed for a patient with endometrial cancer confirmed to have the possibility of lymph node metastasis or high lymph node metastatic potential. On the other hand, it is considered that lymph node dissection does not have to be performed for a patient with endometrial cancer confirmed to have no possibility of lymph node metastasis, low lymph node metastatic potential, or good prognosis.

The testing kit for assessing lymph node metastasis or lymph node metastatic potential, or prognosis of endometrial cancer according to the present invention comprises a testing reagent and/or an assay device for measuring the expression level of the marker in the biological sample isolated from a patient. Specific examples thereof include a reagent for nucleic acid amplification or hybridization comprising an oligonucleotide specifically binding (hybridizing) to the transcription product (mRNA) of the marker, a reagent for immunoassay comprising an antibody recognizing the translation product (protein) of the marker of the present invention, and an immunoassay device in which the antibody is immobilized. The oligonucleotide, the antibody, or the like included in the kit can be obtained by a method known in the art as mentioned above.

The testing kit can further comprise a labeling reagent, a buffer solution, a chromogenic substrate, a secondary antibody, a blocking agent, and equipment or a control necessary for the test, in addition to the antibody or the nucleic acid.

The apparatus for assessing lymph node metastasis or lymph node metastatic potential, or prognosis of endometrial cancer according to the present invention comprises a unit of measuring the expression level of the marker of the present invention, a unit of evaluating or assessing the obtained data, and a unit of outputting the results of evaluation or assessment, and can be arbitrarily configured using respective units known in the art.

### Examples

### Example 1 Discrimination between endometrial cancer metastatic to lymph nodes and endometrial cancer without metastasis

### (1) Specimen

A main lesion was collected during primary lesion harvesting from 7 lymph node metastasis-positive patients and 53 lymph node metastasis-negative patients with endometrial adenocarcinoma G1 and G2 localized to the uterine corpus, under their comprehensive consent before surgery.

Each harvested tissue section was frozen in liquid nitrogen immediately after the harvesting, placed in a 2 mL microtube, and preserved at -80°C.

### (2) Measurement of SEMA3D or TACC2 expression level using RT-qPCR

### 1) Extraction and purification of RNA

The preserved tissue section was placed in divided portions in 2 mL sample tubes such that the amount of the tissue section was 50 mg or less per tube. A zirconia bead φ2.0 and an RNA extraction reagent (RNeasy(R) Mini Kit (Qiagen N.V.)) were added to each tube. The tissue section was homogenized at 13500 rpm for 3 minutes using a bead-type cell lysis apparatus (TOMY Micro Smash MS-100). Next, RNA was extracted according to the protocol of the RNA extraction reagent. The extracted RNA was assayed for ultraviolet absorption (230, 260, and 280 nm) using a spectrophotometer (Thermo Nano Drop), and 260/230 and 260/280 ratios were confirmed to be 1.9 to 2.2.

### 2) Purification of cDNA

1st strand cDNA was synthesized from 2000 ng of the surgically harvested specimen-derived extracted RNA obtained in 1) according to the protocol of PrimeScript(TM) 1st strand cDNA Synthesis Kit (Takara Bio Inc.).

### 3) Measurement of SEMA3D expression level or TACC2 isoform L or S (SEQ ID NO: 3 or 4, respectively) expression level by RT-qPCR

Reaction solutions were prepared according to the protocol of Fast SYBR(R) Green Master Mix (Applied Biosystems, Inc.) and subjected to RT-qPCR using the primers given below and Life Technologies 7500 Fast Real-Time PCR System.
a) SEMA3D primer (TAKARA Primer Set ID: HA239516)
b) SUDS3 primer (TAKARA Primer Set ID: HA237699)
c) TACC2 isoform L or S primer:
   Primer F: CCAgTTgCTgAAgggCAgAA (SEQ ID NO: 8)
   Primer R: gCggACCTTggAgTCTgAg (SEQ ID NO: 9)

Each gene expression level was calculated as -ΔCt by subtracting the Ct value of SUDS3 quantified as a control gene from the Ct value of SEMA3D or TACC2 isoform L or S, followed by multiplication by -1, and assessed for the statistically significant difference between the lymph node metastasis-positive group and -negative group by the unpaired student t-test. The SUDS3 selected as a control gene (housekeeping gene) is a gene confirmed to have small difference in expression level between the lymph node metastasis-positive group and -negative group. PCR amplification efficiency was confirmed to be almost constant in this quantification test for both the genes.

The results are shown in Figures 1 and 2.

### (3) Results

1) The SEMA3D expression level was a significantly higher value in the lymph node metastasis-negative group compared with the lymph node metastasis-positive group (Figure 1, P = 0.0065). The sensitivity and specificity of SEMA3D were further calculated, and a ROC curve was prepared. As a result, AUC was 0.87, indicating that SEMA3D can serve as a biomarker having both high sensitivity and high specificity (Figure 2).
   As seen from these results, SEMA3D can be used as a marker to determine the presence or absence of lymph node metastasis without being limited by early cancer, advanced cancer, and tissue malignancy.
2) The expression level of TACC2 isoform L or S was a significantly higher value in the lymph node metastasis-positive group compared with the lymph node metastasis-negative group (Figure 3, P = 0.0126).
3) As a result of studying -ΔCt (SEMA3D-TACC2 isoform L or S) of the SEMA3D and TACC2 isoform L or S genes in combination, the relative expression level significantly differed between the lymph node metastasis-positive group and -negative group (P value = 0.0004). This -ΔCt indicated low expression in the lymph node metastasis-positive group and high expression in the negative group (Figure 4). Also, the lymph node metastasis-positive group had a low value of -ΔCt (SEMA3D) and a high value of -ΔCt (TACC2), indicating that the expression levels of SEMA3D and TACC2 isoform L or S negatively correlate with each other in diagnosis of lymph node metastasis (Figure 5).

The sensitivity and specificity of SEMA3D and TACC2 isoform L or S were further calculated, and ROC curves were prepared. AUC of -ΔCt (SEMA3D) and -ΔCt (TACC2) of the single genes was as high as 0.87 and 0.78, respectively, whereas AUC of -ΔCt (SEMA3D-TACC2 isoform L or S) was 0.96, and both sensitivity and specificity were better (Figure 6).

These results indicated that the biomarker comprising SEMA3D and TACC2 in combination can be used to more accurately assess the presence or absence of lymph node metastasis without being limited by early cancer, advanced cancer, and tissue malignancy. Accordingly, when a cutoff value is set to the limit at which the false negativity rate is 0% (in Figure 6, a point at which sensitivity is 1.0 and 1-specificity is 0.1 or less), it is expected for endometrial cancer groups that a great majority (92%) of unnecessary lymph node dissection cases can be avoided while a risk of leaving cancer tissues in the body is circumvented.

### Example 2 Validation of localization of SEMA3D or TACC2 (1) Immunohistological staining of SEMA3D protein or TACC2 protein

### 1) Preparation of section sample

A biological sample isolated from each endometrial cancer patient was fixed in formalin by a routine method, then embedded in paraffin, and sliced into a 3 µm tissue section, which was attached to slide glass. The resultant was used as a section sample.

### 2) Immunohistochemical staining

After deparaffinization of each section, the section for TACC2 protein-antibody reaction was autoclaved at 120°C for 10 minutes in a 0.01 M (pH 6.0) citrate buffer solution for antigen retrieval. The section for SEMA3D protein-antibody reaction was heated twice with 700 W microwave for 6 minutes in a 0.01 M (pH 6.0) citrate buffer solution for antigen retrieval.

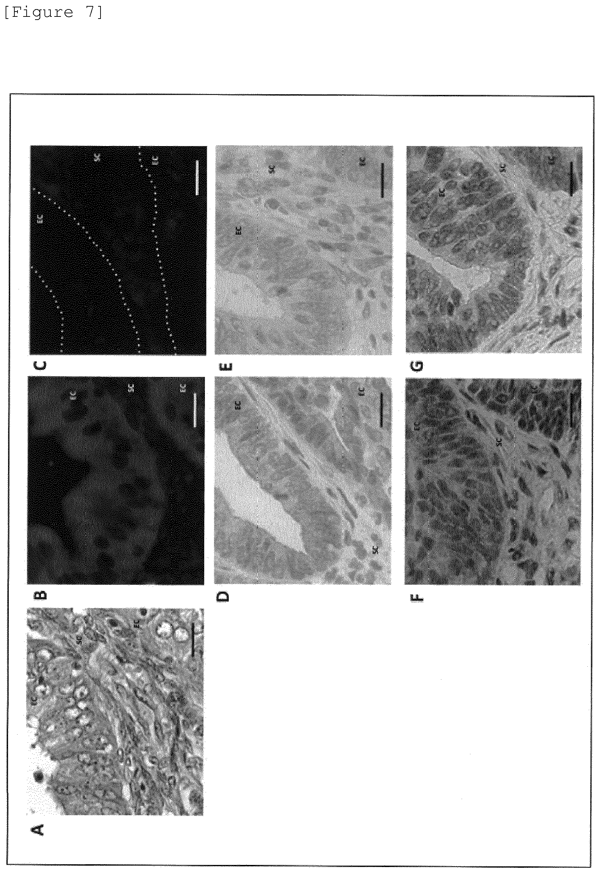

Each section was reacted with a 3% hydrogen peroxide solution for 10 minutes to block peroxidase. For primary antibody reaction, the section was incubated at 4°C with polyclonal TACC2 antibody (Merck Millipore, #2397077) or polyclonal SEMA3D antibody (Sigma-Aldrich Co. LLC, #HPA037522-100UL) diluted 2000-fold with PBS containing 10% goat serum. In negative control experiments, rabbit IgG (DAKO, #1 X0936) was used as a primary antibody. Secondary antibody reaction was performed using the labeled streptavidin-biotin method. The antigen-antibody complexes were visualized using a 3,3-diaminobenzidine solution (1 mmol/L 3,3-diaminobenzidine, 50 mmol/L PBS buffer solution (pH 7.4), 0.006% H₂O₂) . Nucleus was counterstained with hematoxylin-eosin and observed under a microscope. The results of hematoxylin-eosin staining are shown in Figure 7A. The results of immunostaining the SEMA3D protein are shown in Figure 7D. The negative control for Figure 7D is shown in Figure 7E. The results of immunostaining the TACC protein are shown in Figure 7F. The negative control for Figure 7F is shown in Figure 7G.

### (2) Fluorescent in situ hybridization (FISH) for SEMA3D mRNA

### 1) FISH probe

FISH probes for SEMA3D mRNA (NM_152754) were designed using Stellaris(R) RNA FISH Probe Designer (Biosearch Technologies Inc., Petaluma, CA) (https://www.biosearchtech.com/support/tools/design-software/stellaris-probe-designer) and labeled with carboxyfluorescein. The FISH probes used were a mixture of 25 probes designed for the whole nucleotide sequence of SEMA3D mRNA. The probes used are shown in Table 1.

**[Table 1]**

| Probe name | Probe sequence(5'→3') | SEQ ID NO |
|---|---|---|
| SEMA3D_1 | caaagtgccagtgactggaa | SEQ ID NO: 10 |
| SEMA3D_2 | cacattctgtattggcatct | SEQ ID NO: 11 |
| SEMA3D_3 | tctgccagactccaaattat | SEQ ID NO: 12 |
| SEMA3D_4 | gatgtagtggtggtcatgag | SEQ ID NO: 13 |
| SEMA3D_5 | gccttaagaaaagtcgtcca | SEQ ID NO: 14 |
| SEMA3D_6 | tcataggttttgcttggaca | SEQ ID NO: 15 |
| SEMA3D_7 | cagagtgccgctttatgaaa | SEQ ID NO: 16 |
| SEMA3D_8 | gtacaattgttgctgcttca | SEQ ID NO: 17 |
| SEMA3D_9 | tctctggcaagacaacagtc | SEQ ID NO: 18 |
| SEMA3D_10 | gtcgccatattttacatctt | SEQ ID NO: 19 |
| SEMA3D_11 | gcttgttgggatttaggtat | SEQ ID NO: 20 |
| SEMA3D_12 | gggctttgcagtaatacatc | SEQ ID NO: 21 |
| SEMA3D_13 | ttgtatctcaaccgtgactc | SEQ ID NO: 22 |
| SEMA3D_14 | agattattgtcttgtgcctt | SEQ ID NO: 23 |
| SEMA3D_15 | taaaccccctatttttagga | SEQ ID NO: 24 |
| SEMA3D_16 | tgcatggtaaattccatgct | SEQ ID NO: 25 |
| SEMA3D_17 | tttgtgtctgacctcttatt | SEQ ID NO: 26 |
| SEMA3D_18 | gctacttataccctagtgaa | SEQ ID NO: 27 |
| SEMA3D_19 | ccatgcatattcagagggaa | SEQ ID NO: 28 |
| SEMA3D_20 | gccacagatcttgcattaag | SEQ ID NO: 29 |
| SEMA3D_21 | taattgctttgagctcgcta | SEQ ID NO: 30 |
| SEMA3D_22 | gggattctatttagcagcat | SEQ ID NO: 31 |
| SEMA3D_23 | attcttccactactgttgtg | SEQ ID NO: 32 |
| SEMA3D_24 | agggcatctgacacattttg | SEQ ID NO: 33 |
| SEMA3D_25 | tgctaaccactcacaatacc | SEQ ID NO: 34 |

### 2) Preparation of section sample

A biological sample isolated from each endometrial cancer patient was fixed in formalin by a routine method, then embedded in paraffin, and sliced into a 4 µm tissue section, which was attached to slide glass. The resultant was used as a section sample.

### 3) Fluorescent in situ hybridization (FISH)

Each section was deparaffinized according to the protocol (www.biosearchtech.com/stellarisprotocols, Protocol for Formalin-Fixed, Paraffin-Embedded (FFPE) Tissue) and then incubated at 37°C for 20 minutes in 1 × PBS containing 10 µg/ml proteinase K (Wako Pure Chemical Industries, Ltd., #169-22761).

A tissue section attached to a slide, used as a negative control, was pretreated with 50 µg/ml ribonuclease A (Nacalai Tesque, Inc., #30100-31) at 37°C for 30 minutes before a hybridization step to remove mRNA in the section. For hybridization, the section was reacted at 37°C for 16 hours with a hybridization solution containing 125 nM probes. Nuclear staining with DAPI was performed by a routine method. Fluorescence was observed under AXIOVERT 200M inverted wide-field fluorescence microscope (Zeiss, Oberkochen, Germany).
The results of fluorescence *in situ* hybridization for SEMA3D mRNA are shown in Figure 7B. The negative control for Figure 7B is shown in Figure 7C. In Figure 7B, the SEMA3D mRNA is shown in green. In Figures 7B and 7C, the nucleus stained with DAPI is shown in blue.

### (3) Results

1) The immunostaining of the TACC2 protein exhibited positivity in agreement with endometrial cancer cells and specifically exhibited positivity in agreement with the nuclei of endometrial cancer cells (Figures 7F and 7G).
2) The immunostaining of the SEMA3D protein also exhibited positivity in agreement with endometrial cancer cell cytoplasms, albeit with large specimen errors because this protein has an Ig domain and is extracellularly secreted (Figures 7D and 7E).
3) In order to further identify SEMA3D localization, probes for SEMA3D mRNA were prepared and used in *in situ* hybridization (FISH). As a result, the green fluorescence of the SEMA3D mRNA mainly exhibited positivity in agreement with the cytoplasms of endometrial cancer cells (Figures 7B and 7C).

These results demonstrated that the identified marker is expressed only in endometrial cancer cells, not in stromal cells mixed in the specimens.

### Industrial Applicability

According to the present invention, the determination of the presence or absence of lymph node metastasis of the endometrial cancer or the prediction of its occurrence can be objectively performed by examining primary endometrial lesions collected before or during operation. This can avoid unnecessary lymph node dissection and thus allows complications such as postoperative lymphedema to be decreased.

Specifically, image assessment or tumor markers used in conventional diagnosis of lymph node metastasis are not suitable for early cancer or micrometastasis. By contrast, the marker of the present invention (SEMA3D, or SEMA3D and TACC2) can be used to diagnose lymph node metastasis even in early cancer, and is likely to also be able to detect micrometastasis. If diagnosis of lymph node metastasis by the quantitative analysis of marker gene expression in primary lesions is achieved, cases which can avoid retroperitoneal lymph node dissection can be extracted from metastasis-negative groups in consideration of histological malignancy. This may permit circumvention of complications caused by lymph node dissection surgery or mitigation of operative stress on patients.

Even an endometrial cancer patient who has undergone lymph node dissection in addition to dissection of a primary lesion for a reason such as a high risk may be judged as being lymph node metastasis-negative in final pathological diagnosis. In this case, although the possibility of false negativity based on the accuracy of harvesting of a lymph node metastatic focus and the accuracy of pathological diagnosis cannot be denied, the patient can be confirmed to have a high risk of lymph node metastasis in the presence of low SEMA3D expression or low expression of(SEMA3D-TACC2). Individualized medicine such as addition of postoperative treatment for radical treatment and recurrence prevention or establishment of follow-up intervals can be established according to the risk.

## Claims

1. A method for assessing lymph node metastasis or lymph node metastatic potential, or prognosis of endometrial cancer, comprising a step of measuring an expression level of SEMA3D in a biological sample derived from a cancer tissue isolated from an endometrial cancer patient.

2. The method according to claim 1, wherein the method comprises the following steps a and b:
a) the step of measuring an expression level of SEMA3D in a biological sample derived from a cancer tissue isolated from an endometrial cancer patient; and
b) a step of comparing the expression level of SEMA3D with a control level or a threshold level.

3. The method according to claim 1 or 2, wherein when the expression level of SEMA3D is larger than the control level or the threshold level, it is determined that there is no lymph node metastasis, lymph node metastatic potential is low, or prognosis is good.

4. The method according to any one of claims 1 to 3, wherein the expression level of SEMA3D in the biological sample is an amount of a transcription product or a translation product of SEMA3D in an endometrial cancer tissue.

5. A method for assessing lymph node metastasis or lymph node metastatic potential, or prognosis of endometrial cancer, comprising a step of measuring expression levels of SEMA3D and TACC2 in a biological sample derived from a cancer tissue isolated from an endometrial cancer patient.

6. The method according to claim 5, wherein the method comprises the following steps c and d:
c) the step of measuring expression levels of SEMA3D and TACC2 in a biological sample derived from a cancer tissue isolated from an endometrial cancer patient; and
d) a step of comparing the expression levels of SEMA3D and TACC2 with a control level or a threshold level.

7. The method according to claim 5 or 6, wherein when a relative expression level of the expression level of SEMA3D compared with the expression level of TACC2 is larger than the control level or the threshold level, it is determined that there is no lymph node metastasis, lymph node metastatic potential is low, or prognosis is good.

8. The method according to any one of claims 5 to 7, wherein the expression levels of SEMA3D and TACC2 in the biological sample are amounts of transcription products or translation products of SEMA3D and TACC2 in an endometrial cancer tissue.

9. A testing kit for assessing lymph node metastasis or lymph node metastatic potential, or prognosis of endometrial cancer for use in a method according to any one of claims 1 to 4, the testing kit comprising an antibody specifically binding to SEMA3D protein, or an oligonucleotide specifically recognizing a nucleic acid derived from SEMA3D gene.

10. A testing kit for assessing lymph node metastasis or lymph node metastatic potential, or prognosis of endometrial cancer for use in a method according to any one of claims 5 to 8, the testing kit comprising an antibody specifically binding to SEMA3D protein and an antibody specifically binding to TACC2 protein, or an oligonucleotide specifically recognizing a nucleic acid derived from SEMA3D gene and an oligonucleotide specifically recognizing a nucleic acid derived from TACC2 gene.

11. An apparatus for assessing lymph node metastasis or lymph node metastatic potential, or prognosis of endometrial cancer for use in a method according to any one of claims 1 to 8.

12. Use of SEMA3D as a marker for assessing lymph node metastasis or lymph node metastatic potential, or prognosis of endometrial cancer.

13. Use of SEMA3D and TACC2 as markers for assessing lymph node metastasis or lymph node metastatic potential, or prognosis of endometrial cancer.
